(19)

Europäisches
Patentamt
European
Patent Office
Office européen
des brevets

(11) **EP 4 036 115 A1**

(12) **EUROPEAN PATENT APPLICATION**
published in accordance with Art. 153(4) EPC

(43) Date of publication:
**03.08.2022 Bulletin 2022/31**

(21) Application number: **20855201.8**

(22) Date of filing: **20.08.2020**

(51) International Patent Classification (IPC):
*C07K 16/28* (2006.01)    *C07K 16/46* (2006.01)
*A61K 39/395* (2006.01)    *A61P 35/00* (2006.01)

(86) International application number:
**PCT/RU2020/050195**

(87) International publication number:
**WO 2021/034227 (25.02.2021 Gazette 2021/08)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **21.08.2019 RU 2019126380**

(71) Applicant: **Joint-Stock Company "Generium" Petushinskiy rayon, Vladimir Region, 601125 (RU)**

(72) Inventors:
• **PISKUNOV, Aleksandr Aleksandrovich**
  **Vladimirskaya obl., 601125 (RU)**
• **ABBASOVA, Svetlana Georgievna**
  **Vladimirskaya obl., 601125 (RU)**

• **MOROZOV, Anton Nikolaevich**
  **Moscow, 127549 (RU)**
• **SHUSTER, Aleksandr Mikhailovich**
  **Moscow, 115184 (RU)**
• **SLAVNY, Peter**
  **London Road Cambridge CB22 3EG (GB)**
• **GRIFFITHS, Daniel**
  **London Road Cambridge CB22 3EG (GB)**
• **KACZYNSKA, Izabela**
  **London Road Cambridge CB22 3EG (GB)**
• **MCCAFFERTY, John**
  **London Road Cambridge CB22 3EG (GB)**
• **DYSON, Michael**
  **London Road Cambridge CB22 3EG (GB)**

(74) Representative: **Studio Torta S.p.A.**
**Via Viotti, 9**
**10121 Torino (IT)**

(54) **COMPLEMENTARITY-DETERMINING REGIONS FOR BINDING CD3, AND BISPECIFIC ANTIGEN-BINDING MOLECULE CONTAINING SAID CDRS**

(57)    The invention relates to the field of immunology and biotechnology, and also to the creation of novel effective high-molecular compounds having therapeutic properties. Variants of amino acid sequences of complementarity-determining regions (CDR) of antigen-binding fragments of a protein molecule specific to human cell CD3 receptors are proposed. The proposed CDR can be used for engineering various antigen-binding molecules, one of the essential function of which is a specific interaction with CD3-bearing cells. Bispecific molecules that specifically bind to human cell CD3 and CD19 receptors were engineered by means of the inclusion of said CDR in the composition of an antibody-like molecule intrinsically binding to the CD19 receptor. The proposed CDR can be used for engineering bispecific therapeutic antibodies that are suitable for creating a drug for treating cancer, in particular for treating haematological diseases associated with B-cell malignancies, such as acute lymphoblastic leukemia and B-cell lymphomas, including the refractory and recurrent forms thereof.

| Bottom | 1.015 |
|---|---|
| Top | 30.71 |
| LogEC50 | -0.5041 |
| HillSlope | 1.149 |
| EC50 | 0.3132 |
| R square | 0.9858 |

Fig. 1A

EP 4 036 115 A1

# EP 4 036 115 A1

## Description

The invention relates to the field of immunology and biotechnology, and also to the creation of novel effective high-molecular compounds having therapeutic properties. Variants of amino acid sequences of complementarity-determining regions (CDRs) of antigen-binding fragments in a protein molecule specific to the human cell CD3 receptor are proposed. The proposed CDR sequences providing binding of these fragments to the CD3 antigen were produced with the use of the phage display technique. The proposed CDRs may be used for engineering various antigen-binding molecules an essential function of which is a specific interaction with CD3-bearing cells. When these CDRs are included into a composition of an antibody-like molecule initially binding to the CD19 receptor, bispecific molecules are also constructed that specifically bind to the human cell CD3 and CD19 receptors. In one embodiment, bispecific molecules are composed of two polypeptide chains, each of them comprising a light chain variable domain specific to the CD3 antigen (VL CD3), a heavy chain variable domain specific to the CD19 antigen (VH CD19), a light chain variable domain specific to the CD19 antigen (VL CD19), and a heavy chain variable domain specific to the CD3 antigen (VH CD3). The domains are arranged in the following order: VL CD3 - VH CD19 - VL CD19 - VH CD3. This arrangement of variable domains in an antigen binding molecule from the N-terminus to the C-terminus of a polypeptide provides the possibility of binding such a molecule to two different antigens simultaneously, which determines its therapeutic effectiveness due to selective binding to an extracellular portion of the human CD3 and CD19 receptors expressed by T- and B-cells, respectively. When a molecule binds to extracellular portions of the CD3 and CD19 receptors simultaneously, a contact between T- and B-cells takes place, which results in activation of T-cells, formation of a cytolytic synapse, and release of proteolytic enzymes destroying CD19-positive target cells. The proposed structure of arrangement of antigen-binding region of a bispecific molecule enables to form a molecule that effectively binds the extracellular portions of the CD3 and CD19 receptors expressed by cells of various kinds. Such a molecule activates T-cells in response to interaction with target cells expressing a tumor antigen (CD19), which results in their cytolysis. The proposed group of inventions enables, on the basis of a CDR combination contained in any of the proposed fourteen antigen-binding fragments, to produce a recombinant monoclonal bispecific therapeutic antibody suitable for creation of a medicine for treating tumor diseases, in particular haematologic diseases associated with B-cell malignancies.

Abbreviations and terms:

> PBMCs - Peripheral blood mononuclear cells
> TDCC - T-cell-dependent cytotoxic activity
> BSAB - bispecific antibody
> MFI - mean fluorescence intensity
> V - variable domain
> VL - light-chain variable domain
> VH - heavy-chain variable domain
> CDR - complementarity-determining region
> Fc-fragment - Fc, fragment crystallizable region, Fc region
> Antibody - molecule of immunoglobulin for which an antigen exists

Affinity - thermodynamic characteristic that quantitatively described a force of interaction between an antigen and an antibody; it may be defined, according to the law of mass action, as a concentration ratio of an antigen-antibody complex to the product of component concentrations.

Avidity - characteristic of total stability of an antigen-antibody complex; it is determined by affinity of an antibody to an antigen, by a number of antigen-binding sites in an antibody molecule, and by peculiarities of an antigen spatial structure creating steric hindrances for forming a complex. Unlike affinity that is a thermodynamic parameter quantitatively describing a force of a single interaction between an antigen and an antibody, avidity describes a force of cooperative affine interactions.

Antigen-binding region of an antibody - an antibody portion recognizing an epitope. It has several names: active or antigen-binding site of antibodies, antideterminant, or paratope.

Bispecific antibodies - immunoglobulin molecules that combine antigen-binding regions for two different antigens in one molecule. A bispecific antibody is capable of binding two antigens simultaneously.

In this invention, B-cell malignancies mean:

- B-cell precursor tumors
- Precursor B-lymphoblastic lymphoma / leukemia (B-cell precursor acute lymphoblastic leukemia)

- B-cell tumors with phenotype of mature lymphocytes

  1. Chronic lymphocytic leukemia / lymphocytic lymphoma
  2. B-cell pro-lymphocytic leukemia
  3. Lymphoplasmocytic lymphoma
  4. Splenic marginal zone lymphoma
  5. Hairy cell leukemia
  6. Myeloma or plasmacytoma (solitary and extraosseous)
  7. Extranodal marginal zone B-cell lymphoma of mucosa-associated lymphoid tissue (MALT-lymphoma)
  8. Nodal marginal zone B-cell lymphoma (with or without monocytoid B-cells)
  9. Follicular lymphoma
  10. Mantle cell lymphoma
  11. Diffuse large B-cell lymphoma
  12. Mediastinal large B-cell lymphoma
  13. Primary lymphoma of serous body cavities
  14. Burkitt lymphoma / leukemia

B-cell depletion - reduction in a number of cells expressing a certain antigen.

Antibody complementarity - ability of an antibody to specifically interact with an antigen. With rare exception, an antibody interacts only with the antigen that induced its formation and matches it by a spatial structure.

Complementarity-determining region - a hypervariable region of a variable chain, which forms an antigen-binding site of an antibody molecule.

Tumor antigens - antigens produced by cancer cells and capable of invoking a body immune response. They are a result of manifestation of a changed genome of a cancer cell.

Acute lymphoblastic leukemia - a malignant disease of the hematopoietic system, which is characterized by uncontrolled proliferation of immature lymphoid cells (lymphoblasts).

B-cell lymphoma - a variety of non-Hodgkin's neoplasms. This disease appears at the background of uncontrolled division of B-lymphocytes recognizing body cells and tissues as alien ones.

Specificity - ability of an antibody to exhibit different affinity in relation to different antigens.

Epitope, or antigen determinant - a portion of an antigen macromolecule, a small region of an antigen molecule, which interacts with an antibody specific thereto. This region is recognized by cells or molecules of the immune system (i.e. by antibodies, B-lymphocytes, T-lymphocytes).

CD3 (Cluster of Differentiation 3) - a multiprotein complex on the surface of T-lymphocytes; in mammals, it is formed by 4 sub-units: CD3$\gamma$, CD3$\delta$ and two CD3$\epsilon$.

CD19, or B-lymphocytic antigen CD19 - a protein located on the surface of B-lymphocytes.

All the terms and abbreviations, which are used in the present application, have the meaning applicable in the state of the art and are understood by persons skilled in the art. The above definitions are presented for removing any discrepancies.

Brief Description of the Drawings

Fig. 1. Effectiveness of the BSAB preparation binding to human CD19-positive cell lines: A) Namalwa (ATCC® CRL-1432™) cell line; B) Daudi (ATCC® CCL-213™) cell line; C) NALM6 (ATCC® CRL-3273™) cell line; D) Ramos (ATCC® CRL-1596™) cell line; E) Toledo (ATCC® CRL-2631™) cell line; F) Raji (ATCC® CCL-86™) cell line.

Fig. 2. Effectiveness of the BSAB preparation binding to CD3-positive cell lines: A) HUT78 (ATCC® TIB-161™) cell line; B) CCRF-CEM (ATCC® CCL-119™) cell line; C) Jurkat (ATCC® TIB-152™) cell line.

Fig. 3. Interaction between CD19-positive PBMCs of conditionally healthy donors and a FITC-conjugated antibody directed against an extracellular portion of the human CD19 receptor (HD37 clone) (Millipore milli-mark, Cat. No. FCMAB184F) in the presence of the BSAB preparation in different concentrations.

Fig. 4. Interaction of the BSAB with CD3-positive PBMCs in conditionally healthy donors.

Fig. 5. Effectiveness of the human Raji line CD19-positive cell cytolysis by the BSAB preparation when activated PBMCs of conditionally healthy donors are used as effector cells.

Fig. 6. BSAB capability to bind CD3 and CD19 simultaneously when cells of the human Raji line and effector cells of the Jurkat-LUCIA-NFAT (Promega) reporter line are used.

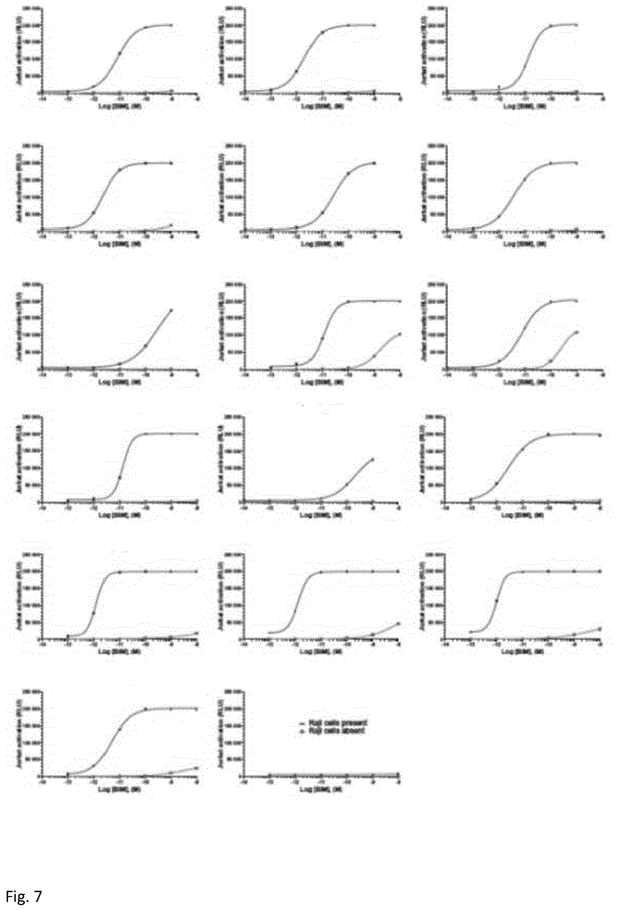

Fig. 7. Evaluation of activity and specificity of bispecific IgG-like molecules in the course of analysis of activation of Jurkat line cells. IgG-like molecules were titrated, and their ability to activate NFAT transcription in reporter cells of the Jurkat line was evaluated. Blue lines indicate activation of effector cells (modified by genetic engineering of Jurkat line cells) in the presence of target cells (Raji line cells); red lines indicate activation of effector cells in the absence of target cells. Clones are numbered from right to left and from top to bottom: 309_ 01_A02; 309_01_G04; 309_01_G12; 309_02_A08; 309_02_F01; 309_04_A04; 309_05_B08; control1; control2; 400_01_D06; 400_03_H07; 410_01_C03; 410_01_C09; 418_01_C05; 418_03_E12; 418_03_H01; negative control.

Fig. 8. Analysis of binding of IgG-like antibodies directed against CD3 to Jurkat line cells by the flow cytometry technique. Y-axis indicates mean fluorescence intensities, X-axis shows logarithms of bispecific IgG-like molecule concentration. Data obtained in independent experiments are shown separately (upper and lower panels). Designations: MFI (FU)- mean fluorescence intensity (fluorescence units). IBC Generium BiM- bispecific IgG-like molecule, positive control, and BiM - bispecific IgG-like molecule, negative control.

Fig. 9. Comparative capability of the 309-02-F08 bispecific antibody of the invention and the BIMSA2 therapeutic antibody to simultaneously bind CD3 and CD19 with the use of human Raji line cells and effector cells of the Jurkat-LUCIA-NFAT (Promega) commercial reporter line.

Fig. 10 A-F. Effectiveness of the human Raji line CD19-positive cell cytolysis by preparations of BSAB variants when activated PBMCs of conditionally healthy donors were used as effector cells. A: clone 309_01_G04; B: clone 309_01_A02; C: clone 309_02_F01; D: clone 309_01_G12; E: clone 309_02_A08; F: clone 309_05_B08.

Fig. 11. Scheme of BSAB possible folding.

Antibodies, also known as immunoglobulins (Ig), are soluble glycoproteins of blood and tissue liquid. Classic antibodies are multimeric proteins combining two identical heavy (H) chains and two identical light (L) chains. H-chains are composed, in their turn, of a variable domain (VH) and three constant (CH1, CH2, CH3) domains and a hinge region between CH1 and CH2 domains. L-chains consist of a variable domain, VL, and a constant domain, CL. These four chains are combined into a molecule by means of non-covalent and covalent (disulfide) bonds. A combination of complementarity determining regions (CDR) of a heavy chain variable domain and a light chain variable domain forms an antigen-binding region, while antibody framework regions (FR) of variable domains and constant domains are not directly involved in recognizing an antigen. Variable regions, though they are located at a great distance from each other in the primary structure of a light chain, become arranged in close proximity to each other when a three-dimensional structure is formed. In the spatial structure of variable domains, hypervariable sequences are located in the area of polypeptide chain folds, which is directed toward corresponding regions of a V-domain of another chain (i.e. CDRs of a light chain and a heavy chain are directed toward each other). As a result of interaction of a variable domain of Hand L-chains, an Ig antigen-binding region (active site) is formed. The spatial structure of this cavity, which is conditioned by the construction of hypervariable regions, determines the capability of antibodies to recognize and bind specific molecules on the basis of spatial conformity (antibody specificity). Hypervariable regions of V-domains do not enter into the composition of an active site completely - an antigen-binding region surface covers app. 30 per cent of a CDR only. Individual amino acid residues in a CDRh interact with antigen epitopes specifically.

A single-chain structure where variable domains of a heavy chain and a light chain are connected by a linker sequence (scFv) is a minimized derivative of an antigen-binding fragment of classic antibodies. An antigen-binding region has a different spatial configuration for each antibody specificity to an antigen. When an antigen contacts an antibody, multiple prosthetic groups of the antigen are in mirror-like correspondence to similar groups of the antibody, owing to which fast and close binding is effected between the antibody and the antigen. If an antibody is highly specific, binding between an antibody and an antigen may be effected by means of several interaction forms at once, such as hydrophobic bonds, hydrogen bonds, ion attraction, Van der Waals forces. An antibody having two variable regions for attaching an antigen is termed bivalent. A bispecific antibody is capable of binding two different antigens simultaneously. Depending on a practical task, developers may change antibody sizes, specificity, affinity, valency, reduce immunogenicity, optimize pharmacokinetic properties and effector functions. In addition, antibodies are produced in the form of recombinant combined proteins comprising antibodies of another specificity, cytokines, protein toxins and radioisotopes, enzymes, fluorescent proteins (see: Deyev S.M. and Lebedenko Ye.N. "Modern technologies for creating non-natural antibodies for clinical use" Acta Naturae (Russian-language version), V. 1, No. 1, 2009, pp. 32-50). Each antibody has unique specificity and high affinity (KD $10^{-8}$-$10^{-11}$ M) to its antigen, which is based on complementarity of an antigen-binding region of such antibody to a certain region of an antigen molecule (epitope). Various antibodies are capable of bind one, two or more antigens simultaneously. Affinity of antibodies used for therapy at present is in a range of nanomolar concentrations (from $10^{-8}$ to $10^{-10}$ M) that are optimal for most tasks. A molecule size of a therapeutic antibody is an important factor determining a possibility of their excretion through kidneys and, respectively, speed of their excretion from a body. Half-time of excretion of proteins through kidneys correlates with a molecule size. An optimal surface charge for therapeutic antibodies is a range of their isoelectric points from 5 to 9. An increase in both positive and negative charge worsens antibody binding to target cells.

The properties of 14 functional antibodies proposed in this invention, that are capable of binding the CD3 receptor complex on the surface of cells expressing it are shown in Table 1. The OKT3 antibody is included as the standard control. The antibodies comprise one of CDR combinations proposed in the invention, and an antigen-binding fragment for the CD19 receptor. The Table shows information about light and heavy chain CDR3 sequences characterizing the antibody, activity according to an analysis of interaction with cells and the industrial production suitability indicator, such as expression productivity. The sequences of all the antibodies are characterized by unique combinations of heavy-chain and light-chain CDR3 sequences and by capability of activating Jurkat line cells (with a reporter gene regulated by the nuclear factor of activated T-cells (NFAT)) in response to interaction with target cells expressing CD19. In the absence of target cells, non-specific activation of Jurkat line cells was low or was not found (at concentrations of bispecific JgG-like molecules ≥ 1 nM). Data on activity with regard to Jurkat line cell activation is shown in Table 1. Binding to a native CD3 on the surface of Jurkat line cells was measured by the flow cytometry technique.

**Table 1. Properties of the proposed functional antibodies capable of binding the CD3 receptor complex on the surface of cells expressing it**

| Clone name | Sequence of heavy-chain CDR3 | Sequence of light-chain CDR3 | Activity ($EC_{50}$ for activation of Jurkat line cells, pM) | Binding to Jurkat line cells | Expression productivit y (mg/L) |
|---|---|---|---|---|---|
| 309_01_A02 | SSRIQAGMDV | QSYDSNNRV | 7.9 | ++ | 15.5 |
| 309_01_G04 | EDYYGGYFDY | QSYDSSNQAV | 2.0 | ++ | 33.2 |
| 309_01_G12 | PSRYSNYVD | QSYDNTNPWV | 13.8 | + | 19.7 |
| 309_02_A08 | TGKNYYYYGMDA | QSYDTSNHVV | 2.2 | ++ | 15.1 |
| 309_02_F01 | DKRYSSGWYEN | QSYDSSSVI | 32.2 | + | 14.9 |
| 309_04_A04 | ATRNGVFDY | QSYDSGVV | 3.7 | ++ | 5.5 |
| 309_05_B08 | STVTRPDFDY | QSYDSSNQRV | 275.8 | + | 8.6 |
| 400_01_D06 | DRGDYGDITYFDY | QSYDSSDRVV | 17.5 | + | 9.3 |
| 400_03_H07 | SGIAAAGFDY | QSYDSSNRV | 328.5 | + | 11.5 |
| 410_01_C03 | ATRNGAFDY | QSYDSSNVV | 3.0 | + | 6.8 |
| 410_01_C09 | PSRYSNYVD | QSYDSRWIWV | 2.2 | + | 5.9 |
| 418_01_C05 | DKPLSGYAFDI | QSYATRTQGVV | 1.5 | + | 2.4 |
| 418_03_E12 | RSFSSVFADY | QSYATRSQSVV | 1.0 | + | 8.3 |
| 418_03_H01 | VYSSGWFSY | QSYDGSNWV | 4.3 | + | 7.2 |
| OKT3 (BiM8) | | | 10.6 | +++ | 0.5 |

During simultaneous interaction of a bispecific molecule with an extracellular portion of the CD3 and CD19 receptors, a contact between T- and B-cells takes place, which results in activation of T-cells, formation of a cytolytic synapse, and release of proteolytic enzymes destroying CD19-positive target cells. At present, the CD3-CD19-directed bispecific antibodies are developed primarily for therapy of B-cell malignancies or for depletion of B-cells, e.g., for therapy of a non-Hodgkin's lymphoma, acute lymphoblastic leukemia, etc. Bispecific antibodies (BSAB) perform the function of adapters that physically connect T-cells with tumor CD19-positive target cells and start their cytolysis. The CD3 is the pan-T-cell receptor, i.e., it is represented on all T-lymphocytes; therefore, when a bispecific antibody is used, various subpopulations of T-cells will be attracted to tumor cells. The CD19 receptor can be stably and uniformly expressed by B-lymphocytic cells (including tumor cells) only. The CD19 is synthesized practically nowhere, except for B-cells, and, hence, provided therapy is specific. The BSAB connects a T-cell and a cancer cell due to simultaneous binding both to a CD3 and to a target antigen. In such a case, T-cells are activated and proliferate, a cytolytic synapse is formed with release of cytotoxic granules, and cytokines are secreted. Directed lysis of cancer cells comprises perforation of a target cell membrane with the involvement of a channel-forming protein, followed by apoptosis induced by granzymes. BSAB binding to a T-cell only, in the absence of a target cell, does not result in T-cell activation.

Examples of bispecific antibodies, which are known in the art (Blood, (ASH Annual Meeting Abstracts), 2009, 114:840), evidence the fact that effective therapeutic concentration of preparations produced on the basis of using multivalent antibodies is significantly lower in comparison to classic antibody-based anti-cancer medicines. It contributes to good tolerance, possibility of providing long courses of treatment, while reducing a risk of side effects.

At present, various polypeptides that are specific with regard to CD19 and CD3, and their use as a component of a medicine for depletion of B-cells and treatment of B-cell diseases are known. The invention described in RU 2228202 discloses a single-chain multifunctional polypeptide specific to CD19 and CD3 antigens with the formula: $V_L$CD19 - $V_H$CD19 - $V_H$CD3 - $V_L$CDS, a polynucleotide encoding this polypeptide, and expressing vector, a cell culture strain, a method for producing this polypeptide, a pharmaceutical composition for treatment of B-cell malignancies, a diagnostic composition< a method of identifying activators or inhibitors of T-cell activation or stimulation, a method for preparing a pharmaceutical composition intended for treatment of B-cell malignancies, a method for treating B-cell malignancies or depleting B-cells, and a method for suppressing development of a pathological condition. Patent RU 2651776 described a recombinant, monoclonal bispecific antibody comprising two chains connected by disulfide bonds, each of them being composed of the following domains: VL(CD3)-L1-VH(CD19)-L2-VL(CD19)-L3-VH(CD3)-H-CH2-CH3(IgG2), where VL (CD3) and VH(CD3). This antibody can be used for treating haematological diseases associated with B-cell malignancies.

A dimeric antigen-binding molecule of a tandem diabody, which is intended for binding to two different antigens simultaneously, is known in the art and described in RU 2613368. This molecule is composed of two identical polypeptide chains.

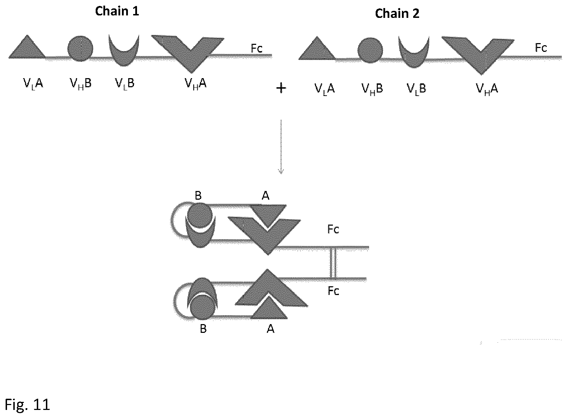

In the proposed invention, each polypeptide chain of the bispecific molecule is composed of a first VL CD3 domain being a light chain variable domain, which is specific to CD3; a second VH CD19 domain being a heavy chain variable domain specific to CD19, a third VL CD19 domain being a light chain variable domain specific to CD19, and a fourth VH CD3 domain being a heavy chain variable domain specific to CD3. These domains are arranged in each of the polypeptide chains in the following order: VLCD3-VHCD19-VLCD19-VHCD3 from the N-terminus to the C-terminus. The first VLCD3 domain is linked to the second VHCD19 domain by means of a first linker L1, the second VHCD19 domain is linked to the third VLCD19 domain by means of a second linker L2, and the third VLCD19 domain is linked to the fourth VHCD3 domain by means of a third linker L3. The linker L2 is composed of 15 or less amino acid residues. When forming the bispecific molecule, the first VLCD3 domain of the first polypeptide chain becomes linked to the fourth VHCD3 domain of the first polypeptide chain with the formation of an antigen-binding site for CD3; the second VHCD19 domain of the first polypeptide chain is linked to the third VLCD19 domain of the first polypeptide chain with the formation of an antigen-binding site for CD19 (Fig. 11). For the illustration purpose the CD3-binding domain is indicated by the A symbol and the CD19-binding domain is indicated by the B symbol in the schematic diagram. The addition of a Fc-region to the molecule prolongs a time of its being in the blood flow due to binding to the neonatal FcRn receptor, thus increasing the excretion half-time of the medicine (Hmila I., et al.. Mol. Immunol. England, 2008, V. 45, No. 14, pp. 3847-3856). The entire molecule is formed from the two chains as a result of the classic antibody folding process. In the process of forming an antibody tertiary structure, disulfide bonds are formed between the constant regions of the heavy chains. One chain is fully functional. A dimer is more stable than a monomer, and a great molecular weight enables it to circulate in the blood longer.

The term "linker", as used in the description of this invention, relates to peptide linkers. A length and/or sequence of a linker influences stability and/or solubility of the bispecific molecule. A linker may improve flexibility of a binding molecule produced and/or may improve its binding to a target antigen by reducing steric obstacles. A linker length and sequence depend on sequences and lengths of sequences linked. A person skilled in the art is well aware of methods for testing suitability of various linkers. For example, properties of a linking molecule may be easily tested by analyzing its linking affinity with the use of various linker types. Stability of a molecule produced may be measured by methods known in the art, such as, for example, high performance liquid chromatography separating a protein material under study into fractions, depending on a protein molecular weight and hydrodynamic characteristics.

More preferable are linkers which are peptides composed of at least 50%, at least 60%, at least 70%, at least 80%, at least 90%, or 100% small amino acids, such as glycine, serine and alanine. Most preferable are linkers composed of glycine and serine molecules only. Preferably, the linker sequences in the invention are composed of 5-15 amino acids. More preferably, the linkers LI, L2, L3 of the antibody directed against CD3-CD19 in the invention are selected from the following sequences:

LI : (XXS)k, where k=2-3, L2: (XXS)n, where $\pi$ =4-5, L3: (XXS)m, where m=2-3, X=G.

According to an embodiment of the invention, the linkers LI, L2, L3 may be represented by sequences corresponding to SEQ ID NO:17 , SEQ ID NO:18, SEQ ID NO:19.

According to another embodiment, each of the above chains is connected by means of a short hinge region to a Fc-sequence of human IgG2. The bispecific molecule, in the absence of a Fc-fragment and a hinge region, also binds to the CD3 and CD19 antigens, does not interact with Fc-receptors due to its low molecular weight, and, therefore is quickly excreted from the blood flow.

A hinge region is a sequence that links a CH1-domain with the CH2-CH3 region of a Fc-fragment in a natural immunoglobulin. The hinge region, as used in the antibody of the invention, is preferably homological to the natural immunoglobulin region and usually comprises cysteine residues linking two heavy chains by means of disulfide bonds, as in natural immunoglobulins. Typical sequences of hinge regions of human and murine immunoglobulins may be found in "Antibody Engineering, a Practical Guide, (Borrebaeck, ed., W. H. Freeman and Co., 1992). Suitable hinge regions of this invention may originate from IgG1, IgG2, IgG3, IgG4 and other immunoglobulin isotypes. In the embodiment provided, the antibody construction of the invention uses the IgG1 hinge region with the sequence presented in SEQ ID NO:15.

Fc-fragment is a terminal portion of an immunoglobulin molecule that interacts with the Fc-receptor on the cell surface and with some proteins of the complement system. Depending on an amino acid sequence of the heavy chain constant region, immunoglobulins are subdivided into classes: IgA, IgD, IgE, IgG and IgM, and some of them may be additionally subdivided into subclasses (isotypes), e.g., IgG1, IgG2, IgG3 and IgG4, IgAl and IgA2. In accordance with constant regions of heavy chains, various immunoglobulin classes are termed [alpha], [delta], [epsilon], [gamma] and [mu], respectively. The four isotypes of human IgG bind various receptors, such as the neonatal Fc-receptor, the activating Fc-receptors gamma, FcgRI, FcgRIIa and FcgRIIIa, the inhibiting receptor FcgRIIb and Clq with different affinity, acquiring various kinds of activities. However, the term "Fc-sequence" in this invention relates, *inter alia,* to Fc-sequences modified for changing affinity to a corresponding receptor. The antibody of this invention comprises an Fc-portion, in order to prolong the half-life. Preferably, such a Fc-sequence has the human origin; more preferably, it is a human Fc-sequence of an IgG antibody, more preferably of IgG2. In one embodiment, this sequence corresponds to SEQ ID NO:16. Such selection of a Fc-sequence of the IgG2 type enables to ensure reduction of effector load conditioned by the Fc-sequence and, thus, reduce directed toxicity of the antibody of the invention.

Thus, the molecule comprising novel antigen-binding regions of the CD3 antigen, proposed in the invention, is constructed on the basis of a single framework, where light- and heavy chain variable domains are connected by three linker regions in a definite order. The molecule acquires additional functional properties owing to an Fc-fragment linked to it by means of a hinge region.

On the basis of the proposed CD3-antigen-binding fragments, the inventors produced a set of antibodies having different sequences of the light- and heavy-chain variable regions for binding CD3 fragments combined into the bispecific molecule according to the above single scheme. A range of antibody-like molecules was produced, which full sequences are given in the list of sequences, Nos. 1-14. The CDR sequences for binding CD19 are created on the basis of a known HD37 antibody, are common for all bispecific antibodies and are - for a heavy chain:

CDRH1: GYAFSSY
CDRH2: WIGQIWPGDGDTNYNGKFKG
CDRH3: RETTTVGRYYYAMDY

and for a light chain:

CDRL1: KASQSVDYDGDSYLN
CDRL2: DASNLVS
CDRL3: QQSTEDPWT

The CDR sequences providing the molecule binding to CD3 antigens are shown in Table 2 for each of the antibodies.

**Table 2. Variants of combinations of CD3-binding regions of light and heavy chain in the composition of the bispecific molecule.**

| Nos. | CLONE | HEAVY CHAIN VARIABLE REGIONS | | | LIGHT CHAIN VARIABLE REGIONS | | |
|---|---|---|---|---|---|---|---|
| | | CDRH1 | CDRH2 | CDRH3 | CDRL1 | CDRL2 | CDRL3 |
| 1 | >309_0 1_A02 | GFTFSSYSM N | SISSSSSYIYY ADSVKG | SSRIQAGMDV | TRSSGTI ASTYVQ | EDNQRPS | QSYDSNNRV |
| 2 | >309_0 1_G04 | GFTVSSNY MS | SAISGSGGST YYADSVKG | EDYYGGYFDY | TRSSGSI ASNYVQ | EDNQRPS | QSYDSSNQAV |
| 3 | >309_0 1_G12 | GYTFTGYY MH | RINPNSGGTN YAQKFQG | PSRYSNYVD | TRSSGSI ASNYVQ | DDDQRP S | QSYDNTNPWV |
| 4 | >309_0 2_A08 | GYTFTGYY MH | IINPSGGSTSY AQKFQG | TGKNYYYYGM DA | TRSSGSI ARNYVQ | EDNQRPS | QSYDTSNHVV |
| 5 | >309_0 2_F01 | GYTFTGYY MH | WINPNSGGT NYAQKFQG | DKRYSSGWYE N | TRSSGSI ASKYVQ | EDNQRPS | QSYDSSSVI |
| 6 | >309_0 4_A04 | GGSFSGYY WS | EISRSGTTNY NSSLKS | ATRNGVFDY | TRSSGSI ASNYVQ | EDNQRPS | QSYDSGVV |
| 7 | >309_0 5_B08 | GFTFSSYAM S | AISGSGGSTY YADSVKG | STVTRPDFDY | TRSSGSI ARNYVQ | EDNQRPS | QSYDSSNQRV |
| 8 | >400_0 1_D06 | GGTFSSYAI S | GIIPIFGTANY AQKFQG | DRGDYGDITYF DY | TGSSGSI ASNYVQ | EDNHRPS | QSYDSSDRVV |
| 9 | >400_0 3_H07 | GFTFSSYSM N | SISSSSSYIYY ADSVKG | SGIAAAGFDY | ARSSGSI ASNYVQ | EDNRRPS | QSYDSSNRV |
| 10 | >410_0 1_C03 | GGSFSGYY WS | EINHSGSTNY NPSLKS | ATRNGAFDY | TRSSGRI ASNYVQ | EDNQRPS | QSYDSSNVV |
| 11 | >410_0 1_C09 | GYTFTSYY MY | RINPNSGGTN YAQKFQG | PSRYSNYVD | TRSSGSI ASNYVQ | EDNQRPS | QSYDSRWIWV |
| 12 | >418_0 1_C05 | GFTFSSYGM H | YINSRSSTIY YADSVKG | DKPLSGYAFDI | TRSSGSI ASNFVQ | DDDQRP S | QSYATRTQGVV |
| 13 | >418_0 3_E12 | GFTFSSYGM H | SISSSSSYIYY ADSVKG | RSFSSVFADY | TRSRGSI ASNYVQ | DDDQRP S | QSYATRSQSVV |
| 14 | >418_0 3_H01 | GGSFSRYY WS | EIKHRGRTN YNPSLKS | VYSSGWFSY | TRSSGSI ARNYVQ | EDNQRPS | QSYDGSNWV |

The activity and specificity of the antibodies produced are confirmed in a number of examples.

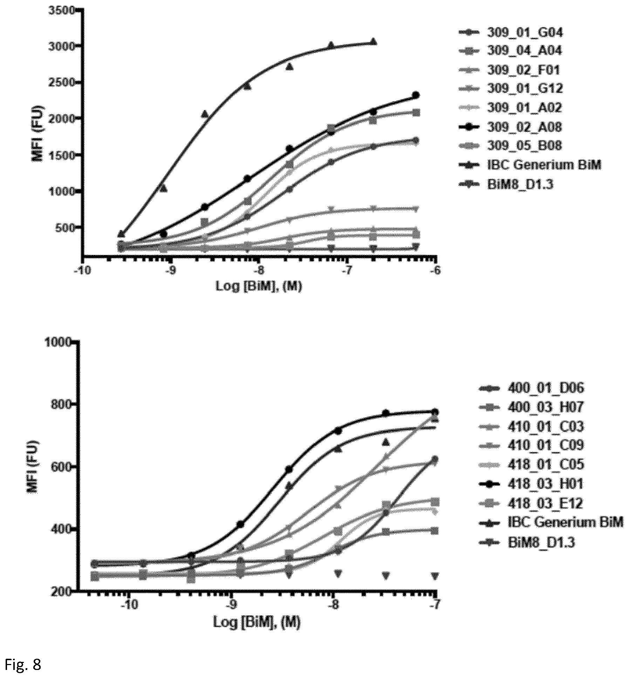

The functionally active bispecific IgG-like molecules were subject to screening for determining their binding to the CD3 endogenous protein present on the surface of the Jurkat line cells in the composition of a T-cell receptor complex. Initially, binding to the Jurkat line cells was evaluated by the flow cytometry technique for one concentration (100 nM) of the bispecific IgG-like molecules; all the fourteen bispecific IgG-like molecules demonstrated binding to the cells. In order to further determine a profile of binding to cells of each clone, the bispecific IgG-like candidate molecules were titrated, and concentration-dependent binding to the Jurkat line cells was evaluated (the results are shown in Fig. 8). These experiments were conducted for two independent series of antibody clones to CD3 separately. In comparison, data are presented on two bispecific IgG-like molecules, used as positive controls, which comprised OKT3 as the fragment binding to CD3 and HD37 binding to CD19. As the negative control, BiM8_D1.3 was used, wherein a single-chain variable fragment (scFv) of an antibody directed against CD3 was substituted by a single-chain variable fragment (scFv) of an antibody directed to lysozyme (D1.3). All the antibodies to CD3 were capable of binding to the native CD3 on the cell surface, though maximum activity and signals were varied significantly among the clones. As can be expected, the control BiM_D1.3 does not bind to the cells in any of the concentration studied.

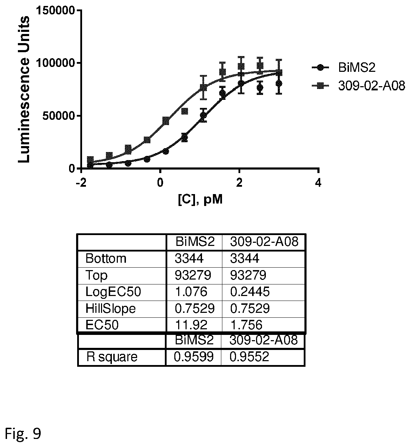

Fig. 9. shows comparative capability of the bispecific antibody of the invention and the therapeutic antibody from Patent RU 2651776 of binding CD3 and CD19 simultaneously with the use of human Raji line cells and effector reporter cells.

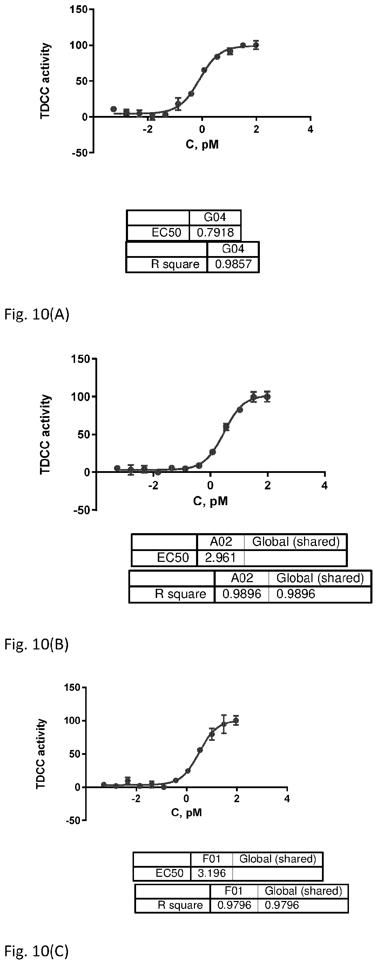

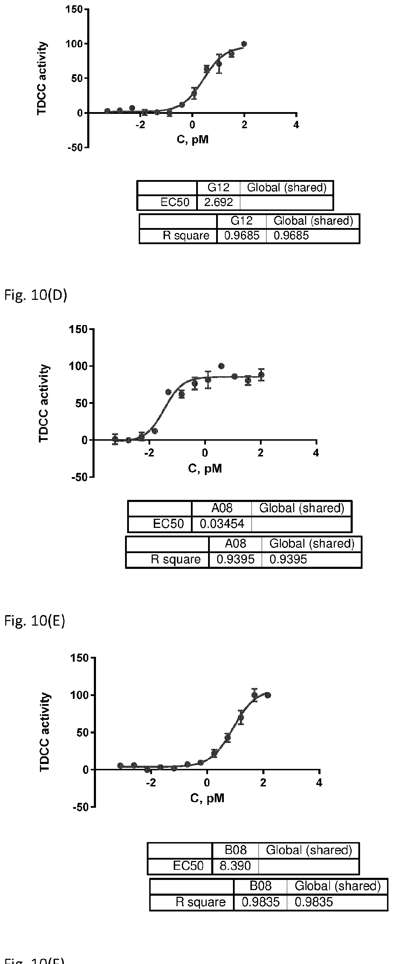

The whole set of the antibodies having the claimed structure was tested for cytolytic activity with regard to CD19-positive target cells by the reporter TDCC-test. The effectiveness of cytolysis of CD19-positive cells of the human Raji line with preparations of proposed BSAB various variants, when activated PBMCs of conditionally healthy donors are used as effector cells, is demonstrated in Fig. 10.

The capability of the bispecific antibodies of causing activation and proliferation of CD3-positive cells in response to contact with CD19-positive target cells and invoking release of antiinflammatory cytokines by T-cells was confirmed.

Thus, the data provided shows that the molecules created with the use of the proposed CDR variants may be efficiently produced in a cell culture, have functional properties not inferior to those of known bispecific CD3- and CD19-directed molecules and, consequently, may be used in developing medicinal preparations used for treating B-cell diseases.

The preparation based on the bispecific molecule, which binding to the CD3 antigen is provided by introducing light and heavy chain variants comprising CDRs listed in Table 2, may be used for therapy of pre-B-cell Philadelphia chromosome-negative recurrent or refractory, acute lymphoblastic leukemia. The mechanism of action is ensured by BSAB binding to the CD19 receptor on the surface of B-lymphocytes and to the CD3-receptor on the surface of effector T-lymphocytes; it initiates T-cell activation that leads to lysis of CD19-positive B-cells.

Specificity and effectiveness of interaction with CD3- and CD19-positive human cell lines were determined for the above antibodies with the use of flow cytometry. The proposed molecules specifically bind to CD3-positive cell lines (Jurkat, HUT-78, CCRF-CEM) and CD19-positive cell lines (Raji, NALM6, Daudi, Namalwa, Ramos, etc.). *In vitro* experiments proved that the molecules of this invention may demonstrate cytotoxic activity that is 7 times higher in comparison to molecules having an alternative domain structure.

For confirming physiological activity of the proposed molecule, the following experiments were conducted: a preparation concentration was determined at which 50% CD3- and CD19-receptors were in complex with the bispecific molecule by using PBMCs of conditionally healthy donors; cytolytic T-cell-mediated activity of the preparation was determined with regard to CD19-positive target cells of human tumor lines with the use of activated PBMCs of conditionally healthy donors as effector cells. The capability of binding CD3 and CD19 simultaneously was confirmed with the use of Raji line cells and those of the Jurkat-LUCIA-NFAT (Promega) commercial reporter line.

Examples 1-4 show that the antibody of the invention has high affinity to target CD3- and CD19-positive human cell lines in the sub-nanomolar range. A concentration at which 50% of free CD3-receptors were identified, when incubated with PBMCs of conditionally healthy donors, varied from 52.1 to 66.5 nM. A concentration at which 50% free CD19-receptors were identified varied from 0.02 to 0.06 nM, depending on a donor. The BSAB preparation exhibited T-mediated cytolytic activity to CD19-positive target cells of the human Raji line when PBMCs of conditionally healthy donors were used as effector cells. EC50 varied from 1.3 pM to 3.1 pM, and percent of maximum lysis of the target cells was from 41 to 100, depending on a donor. In the reporter test, EC50 was 2.3 pM. Both methods confirm simultaneous binding of the 309_02_A08 preparation of the invention to CD3- and CD19-positive cells.

All the molecules of this invention may be produced by using routine methods used at present for producing therapeutic protein molecules with a known primary structure. A gene encoding a polypeptide chain having any one of the proposed amino acid sequences may be produced by firing overlapping chemically synthesized oligonucleotides and then cloned to the pMT1638 expressed vector. The produced pMT1638 vector is verified by restriction mapping and sequencing. After that, transfection is conducted, followed by puromycin resistance selection with the use of standard techniques. Transfection is conducted with the use of the commercially available CHO-K1 strain adapted to suspension growth. The selection of a producer cell line is conditioned by the necessity of forming an optimal glycosylation profile when human proteins are synthesized and by their stability, safety and the possibility of using suspension conditions of culturing this cell line, which are the most important parameters for producing therapeutic antibodies. The producer culturing is conducted in the fed-batch mode with the use of the growth medium BalanCD CHO Growth A (Irvine Scientific) and the nutrient additive BalanCD® CHO Feed 4 (Irvine Scientific) at the temperature of 37°C (Days 1-6), 32°C (Days 7-11) and 5% $CO_2$ in the incubator gas phase for 11 days. After culturing, a cultural liquid is clarified by centrifuging.

Various antibody purification methods may be used for isolating and purifying the target product from the cultural liquid. For example, a scheme may be used that includes an affine step with the use of A-protein-based solvent and high-performance cation-exchange chromatography on sorbent, which provides effective separation of a protein target form and its oligomeric forms.

**Example 1. Determining specificity and effectiveness of BSAB molecule interaction with human CD3- and CD19-positive cells.**

Cell suspensions of CD19-positive cell lines (NALM6 (ATCC® CRL-3273™), Toledo (ATCC® CRL-2631™), Namalwa (ATCC® CRL-1432™), Daudi (ATCC® CCL-213™), Ramos (RA 1) (ATCC® CRL-1596™), Raji (ATCC® CCL-86™)) and CD3-positive cell lines (HuT78 (ATCC® TIB-161™), CCRF-CEM (ATCC® CCL-119™), Jurkat (ATCC® TIB-152™)) with the concentration of $2*10^6$ cells/mL were introduced by 30 μL into a 96-well plate with a V-shaped bottom (Corning Inc, Cat. No. 3894). 30 μL of a 309_02_A08 BSAB clone solution were put into each well in successive 3-fold dilutions from 200 nM to 0.003 nM (final concentrations were from 100 nM to 0.0016 nM) and incubated on ice for 60 minutes. When the incubation was over, the cells were twice washed with the FACS buffer and deposited by centrifuging at 1000 rpM and (4±2)°C. 30 μL of a solution of FITC-tagged antibodies directed against human IgG (Donkey F(ab)2 Anti-Human IgG, Abcam, Cat. No. Ab102437) diluted in the 1:200 ratio were added into each well to the deposited cells, and incubated on ice in the dark for 30 minutes, followed by triple washing with the FACS buffer. Then the cells were fixed by re-suspending the cell deposit in each well in 150 μL of the FIX-buffer (phosphate buffered saline with 1%-solution of formalin). Cell fluorescence intensity in the FITC-channel was measured with the use of the Canto II cytometer.

**Table 3. Effectiveness of BSAB binding to CD19- and CD3-positive cell lines.**

|  | Cell line | EC50, nM |
|---|---|---|
| CD3-positive cell lines | CCRF-CEM | 0.40 |
|  | HUT78 | 1.20 |
|  | Jurkat | 0.37 |
| CD19-positive cell lines | Namalwa | 0.29 |
|  | Daudi | 0.12 |
|  | NALM6 | 0.28 |
|  | Ramos | 0.38 |
|  | Toledo | 0.24 |
|  | Raji | 0.33 |

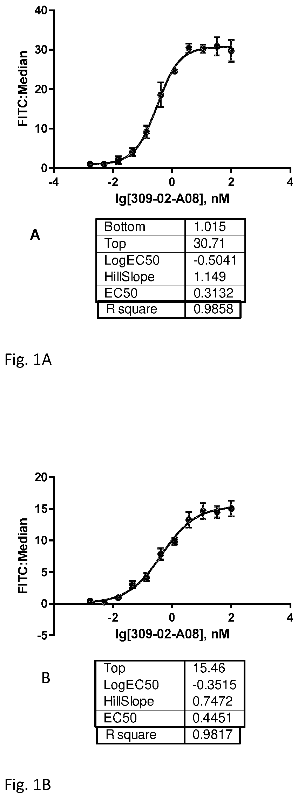

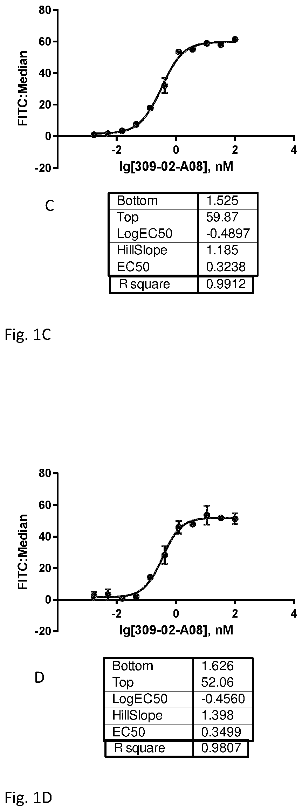

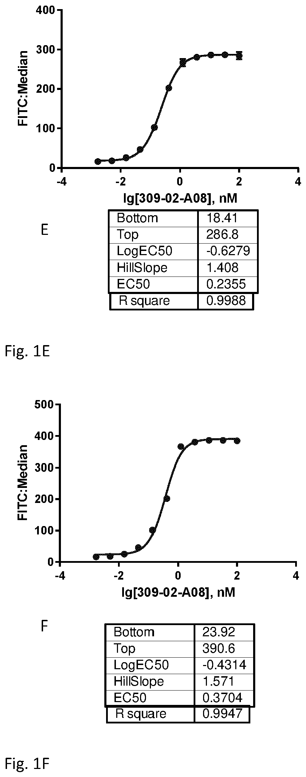

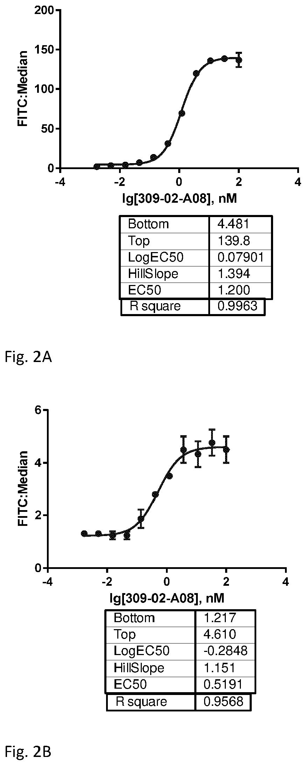

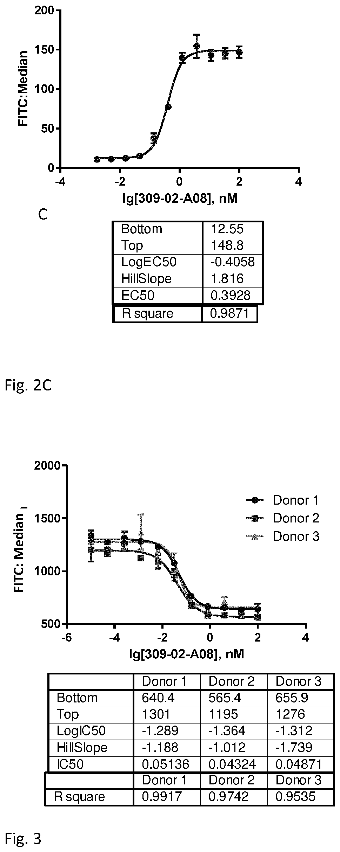

It was shown that the BSAB specifically binds to CD19- and CD3-positive cell lines with sub-nanomolar affinity. The experimental results are demonstrated in Figs. 1 and 2 and Table 3.

**Example 2. Determining a BSAB preparation concentration at which 50% of CD3- and CD19-receptors are in a complex with the antibody, using PBMCs of conditionally healthy donors**

***Isolation of peripheral blood mononuclear cells (PBMCs).***

Blood was taken in sterile conditions from conditionally healthy donors into vacutainers with heparin (Greiner Bio-One, Cat. No. 455084). The blood was diluted with the same volume of the phosphate buffered saline (ECO-Servis, Cat. No. B-60201), layered above Ficoll density gradient (ρ=1.077 g/cm³) ("Pan-Eco", Cat. No. P052) and centrifuged at 800 g and 20±2°C for 30 minutes. PBMCs were collected at the phase boundary and washed twice with an isotonic phosphate buffered saline. A cell deposit was re-suspended in 15 mL of the DMEM/F12 medium (Sigma, Cat. No. D8900-10L) with 10% fetal bovine serum (GE Healthcare, Cat. No. SV30160) and incubated in a cultural vial (SPL Life Sciences Co, Cat. No. 70075) for attaching monocytes at 37±2°C for 4 hours. Upon incubation, unattached cells were collected, a cell suspension was centrifuged, a cell deposit was re-suspended in the FACS buffer with a 1:100 Fc Block solution (BD Pharmingen™, Cat. No. 564220) for inhibiting non-specific binding of Fc-receptors exposed on B-cell membranes. The prepared PBMC suspension with the cell concentration of $4*10^6$/mL was introduced by 50 $\mu$L ($2*10^5$ cells per well) into a 96-well plate with a V-shaped bottom (Corning Inc, Cat. No. 3894).

### BSAB binding to CD19-positive peripheral blood mononuclear cells of conditionally healthy donors.

50 $\mu$L of a 309_02_A08 BSAB clone solution were added to cells in successive 3-fold dilutions in the FACS buffer with the addition of 1:100 Fc Block from 200 nM to 0.003 nM (final concentrations being from 100 nM to 0.0016 nM) and incubated at 37$\pm$2°C for 60 minutes. Upon incubation, the cells were twice washed with the ice-cold FACS buffer, depositing the cells by centrifuging at 1300 rpM and 5$\pm$3°C for 10 minutes. Then, a cell deposit was re-suspended in 100 $\mu$L of the FACS buffer, and FITC-tagged antibodies (Millipore milli-mark, Cat. No. FCMAB184F) were added in the volume of 10 $\mu$L to the extracellular portion of the human CD-19 receptor, final dilution being 1:150, incubated at 5$\pm$3°C for 30 minutes, followed by triple washing. The cells were fixed by re-suspending in 250 $\mu$L of the FIX-buffer (phosphate buffered saline with 1%-solution of formalin). Cell fluorescence intensity in the FITC-channel was measured with the use of the Canto II cytometer. In order to verify data thus obtained, at least 50,000 of events were collected.

### BSAB binding to CD3-positive peripheral blood mononuclear cells of conditionally healthy donors

50 $\mu$L of a 309_02_A08 BSAB clone solution were added to cells in successive 3-fold dilutions in the FACS buffer with the addition of 1:100 Fc Block from 8,700 nM to 0.04 nM (final concentrations being from 2,900 nM to 0.01 nM). Then a solution of biotin-conjugated BSAB with the concentration of 300 nM was prepared and introduced into wells with cells by 50 $\mu$L (preparation final concentration being 100 nM). The cells were incubated at 37$\pm$2°C for 60 minutes. Upon incubation, the cells were twice washed with an ice-cold FACS buffer, depositing the cells by centrifuging at 1300 rpM and 5$\pm$3°C for 10 minutes. The cell deposit was re-suspended in 100 $\mu$L of the FACS buffer, and 10 $\mu$L of the PE-Streptavidin conjugate (BD Pharmingen, Cat. No. 554061) (final dilution being 1:200) was added, followed by incubation at 5$\pm$3°C for 30 minutes and by triple washing. The cells were fixed by re-suspending in 250 $\mu$L of the FIX-buffer (phosphate buffered saline with 1%-solution of formalin). Cell fluorescence intensity in the PE-channel was measured with the use of the Canto II cytometer. In order to verify data thus obtained, at least 50,000 of events were collected.

The obtained data was processed with the use of FlowJo, Excel and GraphPad Prism 6.0 software, the 4-parameter logistic function of dose-depending inhibition, and the "constrain - shared value" function for values of an upper and a lower asymptotes and a sigmoid curve hill slope. A half maximal inhibitory concentration ($IC_{50}$) and a confidence coefficient of function approximation ($R^2$) were determined.

*Results.*

A BSAB concentration, at which 50% of the CD19-receptors were free, and, correspondingly, 50% of the CD19-receptors were in complex with the antibody, was determined as varied from 0.02 to 0.06 nM, depending on a donor (Fig. 3).

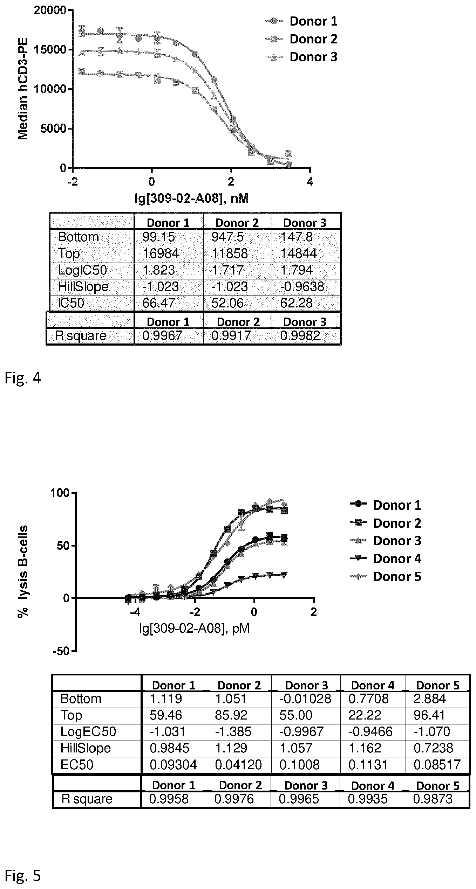

A BSAB concentration, at which 50% of the CD3-receptors were free, and, correspondingly, 50% of the CD3-receptors were in complex with the antibody, was determined as varied from 52.1 to 66.5 nM, depending on a donor (Fig. 4).

**Table 4. BSAB preparation concentration at which 50% of CD3- and CD19-receptors are in a complex with the antibody, determined with the use of PBMCs of conditionally healthy donors**

|  | CD3-receptor | | | CD 19-receptor | | |
|---|---|---|---|---|---|---|
| Donor | GA | PE | KN | PE | KA | AK |
| $IC_{50}$, nM | 66.5 | 52.1 | 62.3 | 0.05 | 0.02 | 0.06 |

### Example 3. Determining T-cell-mediated cytolytic activity of the BSAB with regard to CD19-positive target cells of human tumor lines, when activated PBMCs of conditionally healthy donors are used as effector cells

*Isolation and activation of peripheral blood mononuclear cells (PBMCs).*

PBMCs of conditionally healthy donors were isolated as described in Example 2. IL-2 (Abcam, Cat. No. ab155694) was added to 5 mL of a PBMC suspension with the concentration of $2*10^6$ cells/mL in the DMEM/F12 (Sigma, Cat. No. D8900-10L) medium with 10% fetal bovine serum (GE Healthcare, Cat. No. SV30160) to achieve the final concentration of 5 ng/mL, and Dynabeads magnetic particles with antibodies directed against human CD3 and CD28 receptors in the volume of 65 μL to activate human T-lymphocytes (Gibco, Cat. No. 11131D). The cells were incubated at 37±2°C in the 5.0 ± 0.5% $CO_2$ atmosphere for 5-7 days, counting cell number daily; if necessary, the DMEM/F12 (Sigma Cat. No. D8900-10L) medium comprising 10% of fetal bovine serum (GE Healthcare Cat. No. SV30160) and IL-2 in the concentration of 5 ng/mL were added.

*Scenario of the technique.*

Target cells of the human Raji line (ATCC® CCL-86™) were twice washed to remove the cultural medium with an isotonic phosphate buffered saline, depositing cells by centrifuging at 1300 rpM and 22±2°C for 10 minutes. A cell deposit was re-suspended in the isotonic phosphate buffered saline, 10 μL of 1 mM calcein solution (Molecular Probes, Cat. No. C3100MP) was added in the quantity of 10 μL of the calcein solution for 1 million cells, and incubated for 40 minutes. Upon incubation, the cells were triple washed from free calcein with the isotonic phosphate buffered saline. A density of the cell suspension was brought to $2\times10^5$ cells in 1 mL with the use of the ADCF-Mab medium (GE Healthcare Life Sciences, Cat. No. SH30349.02), and 1/100 of the 100X volume of a probenecid solution (Sigma-Aldrich, Cat. No. P8761-25G) was added.

Activated IL-2/CD3/CD28 T-cells were deposited by centrifuging at 1300 rpM and 22±2°C for 10 minutes, and once washed with the isotonic phosphate buffered saline. A cell deposit was re-suspended in the ADCF-Mab medium, and a cell suspension produced was brought to the concentration of $4\times10^6$ cells in 1 mL.

Twelve successive 3-fold dilutions of the 309_02_A08 BSAB clone from 100 to 0.0005 pM were made, and each solution was introduced in triplicate by 50 μL into wells of a 96-well flat-bottom plate (Corning Inc, Cat. No. 3599). Then, 50 μL of IL-2/CD3/CD28-activated T-cells and 50 μL of Raji cells were introduced into each of the plate wells (T-cells/Raji cell ratio was 10:1). Further, 1) calcein-tagged target cells (control of calcein spontaneous release), 2) calcein-tagged target cells with effector cells (cell lysis conditioned by cytolytic activity of effector cells without addition of the 309_02_A08 preparation), 3) calcein-tagged target cells for subsequent treatment with a Triton X100 solution (Sigma-Aldrich, Cat. No. X100-1L) were introduced into the plate wells. The cells were incubated at 37±2°C for three hours.

In three hours, 20 μL of the Triton X100 solution were added into the wells with the calcein-tagged Raji cells to achieve complete lysis of the cells (MFI_max), and the cells were incubated at 37±2°C for 1 hour.

Upon incubation, the plate was centrifuged at 1300 rpm for 5 minutes, and 120 μL of a supernatant were transferred from each well with the use of a multichannel pipette, this being done carefully in order not to catch cells, into plates made of non-transparent white plastic (Corning Inc, Cat. No. 3912) to measure fluorescence. Fluorescence was measured in a Tecan Infinite M200 plate reader at the excitation wavelength of 488 nm and emission at 518 nm. The obtained data was exported into Excel, and lysis in each experimental well was calculated according to the following formula:

$$\text{Per cent of specific lysis} = (\text{MFIexp} - \text{MFI\_min}) / (\text{MFI\_max} - \text{MFI\_min})*100\%.$$

The obtained data was further processed with the use of GraphPad Prism 6.0 software, the 4-parameter logistic function, and the "constrain - shared value" function for values of an upper and a lower asymptotes and a sigmoid curve hill slope. A half maximal inhibitory concentration ($IC_{50}$) and a confidence coefficient of function approximation ($R^2$) were determined.

*Results.*

The BSAB preparation exhibited cytolytic activity with regard to CD19-positive target cells of the human Raji line when PBMCs of conditionally healthy donors were used as effector cells. EC50 varied from 1.3 pM to 3.1 pM, and target cell maximum lysis percent - from 41 to 100, depending on a donor. The experimental results are shown in Fig. 5.

Table 5. BSAB preparation concentration at which cytolysis effectiveness of 50% CD19-positive cells of the human Raji line was determined when activated PBMCs of conditionally healthy donors were used as effector cells.

|  | BSAB EC50, pM | Maximum lysis, % |
|---|---|---|
| Donor 1 | 2.0 | 41 |
| Donor 2 | 0.13 | 60 |
| Donor 3 | 1.9 | 100 |
| Donor 4 | 1.5 | 95 |

(continued)

|  | BSAB EC50, pM | Maximum lysis, % |
|---|---|---|
| Donor 5 | 1.3 | 48 |

### Example 4. Determining T-cell-mediated BSAB activity with the use of Jurkat-LUCIA-NFAT (Promega) commercial reporter line as effector cells

Description of the technique.

The Raji cell line (ATCC®CCL-86™) were cultured according to the manufacturer's recommendations. Target cells of the human Raji line (ATCC® CCL-86™) were twice washed out of the culture medium with an isotonic phosphate buffered saline, depositing the cells by centrifuging at 1300 rpm and $22\pm2°C$ for 10 minutes. A cell deposit was re-suspended in the test medium (RPMI-1640 without phenol red (Lonza Cat. No. BE12918F) and with 5 % fetal bovine serum (GE Healthcare, Cat. No. SV30160)). Five mL of a cell suspension were prepared with the concentration of $2\times10^5$ cells/mL.

The Jurkat-Luc-NFAT cell line (effector cells) (Promega, Cat. No. jktl-nfat) was defrosted on a water bath at $37\pm2°C$. The content of an ampoule was transferred to a centrifuge tube containing 5 mL of the test medium and centrifuged at 1000 rpm for 5 minutes. A cell deposit was re-suspended in the test medium. Five mL of the cell suspension were made with the concentration of $2.5\times10^6$ cells/mL.

Eleven successive 3-fold dilutions of the 309_02_A08 BSAB clone from 1200 pM to 0.02 pM (final concentrations being from 400 to 0.007 pM) were prepared in the test medium.

40 $\mu$L of preparation solutions were introduced into each A2-H12 well of a U-shaped 96-well culture plate (Corning Inc, Cat. No. 3799) in triplicates, starting from a minimal concentration. 40 $\mu$L of the medium were introduced into each of the A1-H1 wells. The prepared suspensions of Raji and Jurkat-Luc-NFAT cells were mixed and introduced into the A1-H12 wells, 80 $\mu$L into each, of the plate. The plate was incubated at $37\pm2°C$ for 18 hours.

Upon incubation, the content of the plate wells was re-suspended and transferred into the wells, 40 $\mu$L into each, of a 96-well flat-bottom plate made of a non-transparent white plastic (Corning Inc, Cat. No. 3912). Then, 25 $\mu$L of the QUANTI-Luc™ Lucia™ substrate (Invivogen Cat. No. rep-qlc1, rep-qlc2), which was heated to room temperature $(20\pm5°C)$, were put into each well. The content of the wells was stirred carefully. Chemiluminescence intensity was measured in a Tecan Infinite M200 plate reader.

The obtained data was processed with the use of GraphPad Prism 6.0 software. The dependence of chemiluminescence level on a decimal logarithm of the BSAB preparation concentration was approximated with the 4-parameter logarithmic function equation. $EC_{50}$ и $R^2$ were determined automatically.

Results.

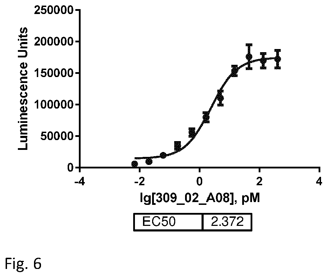

When cells of the Raji line were cultured together with Jurkat-Luc-NFAT cells in the presence of the BSAB, a dose-dependent increase of a chemiluminescence signal was observed; EC50 was 2.3 pM. The experimental results are shown in Fig. 6.

### Example 5. Evaluating functional activity and specificity of the bispecific antibodies by analyzing activation of Jurkat cells

Activation of T-cells was measured with the use of Jurkat line cells with a reporter gene, which were purchased from Promega Co. Data on activity (EC50) of 14 bispecific IgG-like antibodies directed against CD3 is shown in Table 1, and full curves of dose-response dependence are presented in Fig. 7. The studied bispecific IgG-like molecules were titrated and evaluated for their capability of inducing NFAT transcription in effector cells of the Jurkat line in the presence or absence of target cells expressing CD19 (i.e., target-dependent and target-independent activation was evaluated). In this analysis, all the bispecific IgG-like molecules demonstrated high target-dependent activity. Also, data is shown on two different bispecific IgG-like molecules used as positive controls, wherein OKT3 was used as the fragment binding to CD3 (controls 1 and 2). BiM8_D1.3 was used as the negative control (wherein a single-chain variable fragment (scFv) of an antibody directed against CD3 was substituted by a similar fragment of an antibody directed against the D1.3 hen egg lysozyme). In this analysis, Jurkat line cells modified by genetic engineering were used, which stably expressed a luciferase reporter gene regulated by the response element of the nuclear factor of activated T-cells (NFAT). Culturing these reporter cells jointly with CD19-expressing Raji line cells in the presence of functional bispecific antibodies directed against CD3/CD19 results in activation of a T-cell receptor complex and in a luciferase activity increase conditioned by activation of the NFAT response element.

GloResponse™ NFAT-Re-luc2 Jurkat cells (Promega Cat. No. CS 176403), which are ready-to-use after defrosting, were defrosted at a water bath at 37°C for two minutes and carefully transferred to 4 mL of the pre-heated RPMI 1640 medium (Gibco Cat. No. 22400) with the addition of 10% fetal bovine serum (HyClone Cat. No. SH30070.03). 25 $\mu$L of the re-suspended cells were put into each well of a white 96-well plate for analysis (Costar Cat. No. 3917), so the total number of Jurkat line cells in each well was $1 \times 10^5$. A Raji line cell suspension was prepared in the concentration of $2 \times 10^6$/mL in the analysis medium, and 25 $\mu$L of the suspension were added into each well containing reporter cells of the Jurkat line (number of the Raji line cells in each well was $5 \times 10^4$). Further, 5.5 $\mu$L of the bispecific IgG-like molecule (or a solvent used as control, if applicable) under study were added, and the samples were incubated at 37°C for 5 hours (static incubator, 5% $CO_2$). Upon incubation, the plates were left at room temperature (21°C) for 15 minutes, and, then, 55 $\mu$L of Bio-Glo reagent (Promega Cat. No. G7940) were added into each well; the reagent was dissolved in accordance with the manufacturer's instructions). At last, the plates were incubated for 5 minutes (21°C), and luminescence was measured by means of a BMG Pherastar plate reader.

A low level of target-independent activation of Jurkat line cells is a highly promising property of all clones of the proposed bispecific IgG-like molecules.

Sequence listing

<110> Joint-stock company "GENERIUM"
<120> COMPLEMENTARITY-DETERMINING REGIONS FOR BINDING CD3, AND BISPECIFIC
ANTIGEN-BINDING MOLECULE CONTAINING SAID CDRs.
<160> 19


<210> 1
<211> 725
<212> PRT
<213> Artificial sequence
<220>
<223> clone 309_01_A02
<400> 1

DIQLTQTPASLAVSLGQRATISCKASQSVDYDGDSYLNWYQQIPGQPPKLLIYDASNLVSGIPPRFSGSGSGTDFTL
NIHPVEKVDAATYHCQQSTEDPWTFGGGTKLEIKGGSGGAMAEVQLVESGGGLVKPGGSLRLSCAASGFTFSSYSMN
WVRQAPGKGLEWVSSISSSSSYIYYADSVKGRFTISRDNAKNSLYLQMNSLRAEDTAVYYCARSSRIQAGMDVWGQG
TLVTVSSGGGGSGGGGSGGGASNFMLTQPHSVSESPGKTVTISCTRSSGTIASTYVQWYQQRPGSSPTTVIYEDNQR
PSGVPDRFSGSIDSSSNSASLTISGLKTEDAADYFCQSYDSNNRVFGGGTKVTVLGQPAAAGGGGSQVQLQQSGAEL
VRPGSSVKISCKASGYAFSSYWMNWVKQRPGQGLEWIGQIWPGDGDTNYNGKFKGKATLTADESSSTAYMQLSSLAS
EDSAVYFCARRETTTVGRYYYAMDYWGQGTSVTVSSASAAVECPPCPAPPVAGPSVFLFPPKPKDTLMISRTPEVTC
VVVDVSHEDPEVQFNWYVDGVEVHNAKTKPREEQFNSTFRVVSVLTVVHQDWLNGKEYKCKVSNKGLPAPIEKTISK
TKGQPREPQVYTLPPSREEMTKNQVSLTCLVKGFYPSDIAVEWESNGQPENNYKTTPPMLDSDGSFFLYSKLTVDKS
RWQQGNVFSCSVMHEALHNHYTQKSLSLSPGK


<210> 2
<211> 726
<212> PRT
<213> Artificial sequence
<220>
<223> clone 309_01_G04
<400> 2

DIQLTQTPASLAVSLGQRATISCKASQSVDYDGDSYLNWYQQIPGQPPKLLIYDASNLVSGIPPRFSGSGSGTDFTL
NIHPVEKVDAATYHCQQSTEDPWTFGGGTKLEIKGGSGGAMAEVQLVESGGGVVQPGRSLRLSCAASGFTVSSNYMS
WVRQAPGKGLEWVSAISGSGGSTYYADSVKGRFTTSRDNSKNTLYLQMNSLRAEDTAIYYCAKEDYYGGYFDYWGQG
TMVTVSSGGGGSGGGGSGGGASNFMLTQPHSVSESPGKTVTISCTRSSGSIASNYVQWYQQRPGSSPTTVIYEDNQR
PSGVPDRFSGSIDSSSNSASLTISGLKTEDEADYYCQSYDSSNQAVFGGGTKVTVLGQPAAAGGGGSQVQLQQSGAE
LVRPGSSVKISCKASGYAFSSYWMNWVKQRPGQGLEWIGQIWPGDGDTNYNGKFKGKATLTADESSSTAYMQLSSLA
SEDSAVYFCARRETTTVGRYYYAMDYWGQGTSVTVSSASAAVECPPCPAPPVAGPSVFLFPPKPKDTLMISRTPEVT
CVVVDVSHEDPEVQFNWYVDGVEVHNAKTKPREEQFNSTFRVVSVLTVVHQDWLNGKEYKCKVSNKGLPAPIEKTIS
KTKGQPREPQVYTLPPSREEMTKNQVSLTCLVKGFYPSDIAVEWESNGQPENNYKTTPPMLDSDGSFFLYSKLTVDK
SRWQQGNVFSCSVMHEALHNHYTQKSLSLSPGK


<210> 3
<211> 725
<212> PRT
<213> Artificial sequence
<220>
<223> clone 309_01_G12
<400> 3

DIQLTQTPASLAVSLGQRATISCKASQSVDYDGDSYLNWYQQIPGQPPKLLIYDASNLVSGIPPRFSGSGSGTDFTL
NIHPVEKVDAATYHCQQSTEDPWTFGGGTKLEIKGGSGGAMAEVQLVQSGAEVKKPGASVKVSCKASGYTFTGYYMH
WVRQAPGQGLEWMGRINPNSGGTNYAQKFQGRVTMTRDTSISTAYMELSRLRSDDAAVYYCARPSRYSNYVDWGQGT
TVTVSSGGGGSGGGGSGGGASNFMLTQPHSVSESPGKTVTISCTRSSGSIASNYVQWYQQRPGSAPTTVIYDDDQRP
SGVPDRFSGSIDRSSNSASLTISGLKTEDEADYYCQSYDNTNPWVFGGGTKLTVLGQPAAAGGGGSQVQLQQSGAEL
VRPGSSVKISCKASGYAFSSYWMNWVKQRPGQGLEWIGQIWPGDGDTNYNGKFKGKATLTADESSSTAYMQLSSLAS
EDSAVYFCARRETTTVGRYYYAMDYWGQGTSVTVSSASAAVECPPCPAPPVAGPSVFLFPPKPKDTLMISRTPEVTC
VVVDVSHEDPEVQFNWYVDGVEVHNAKTKPREEQFNSTFRVVSVLTVVHQDWLNGKEYKCKVSNKGLPAPIEKTISK
TKGQPREPQVYTLPPSREEMTKNQVSLTCLVKGFYPSDIAVEWESNGQPENNYKTTPPMLDSDGSFFLYSKLTVDKS
RWQQGNVFSCSVMHEALHNHYTQKSLSLSPGK

<210> 4
<211> 728
<212> PRT
<213> Artificial sequence
<220>
<223> clone 309_02_A08
<400> 4

DIQLTQTPASLAVSLGQRATISCKASQSVDYDGDSYLNWYQQIPGQPPKLLIYDASNLVSGIPPRFSGSGSGTDFTL
NIHPVEKVDAATYHCQQSTEDPWTFGGGTKLEIKGGSGGAMAQVQLVQSGAEVKKPGASVKVSCKASGYTFTGYYMH
WVRQAPGQGLEWMGIINPSGGSTSYAQKFQGRVTMTRDTSTSTVYMELSSLRSEDTAVYYCARTGKNYYYYGMDAWG
QGTTVTVSSGGGGSGGGGSGGGASNFMLTQPHSVSESPGKTVTISCTRSSGSIARNYVQWYQQRPGSSPTTVIYEDN
QRPSGVPDRFSASIDSSSNSASLTISGLQTEDEADYYCQSYDTSNHVVFGGGTKLTVLGQPAAAGGGGSQVQLQQSG
AELVRPGSSVKISCKASGYAFSSYWMNWVKQRPGQGLEWIGQIWPGDGDTNYNGKFKGKATLTADESSSTAYMQLSS
LASEDSAVYFCARRETTTVGRYYYAMDYWGQGTSVTVSSASAAVECPPCPAPPVAGPSVFLFPPKPKDTLMISRTPE
VTCVVVDVSHEDPEVQFNWYVDGVEVHNAKTKPREEQFNSTFRVVSVLTVVHQDWLNGKEYKCKVSNKGLPAPIEKT
ISKTKGQPREPQVYTLPPSREEMTKNQVSLTCLVKGFYPSDIAVEWESNGQPENNYKTTPPMLDSDGSFFLYSKLTV
DKSRWQQGNVFSCSVMHEALHNHYTQKSLSLSPGK


<210> 5
<211> 726
<212> PRT
<213> Artificial sequence
<220>
<223> clone 309_02_F01
<400> 5

DIQLTQTPASLAVSLGQRATISCKASQSVDYDGDSYLNWYQQIPGQPPKLLIYDASNLVSGIPPRFSGSGSGTDFTL
NIHPVEKVDAATYHCQQSTEDPWTFGGGTKLEIKGGSGGAMAEVQLVQSGAEVKKPGASVKVSCKASGYTFTGYYMH
WVRQAPGQGLEWMGWINPNSGGTNYAQKFQGRVTMTRDTSISTAYMELSSLRSEDTAVYYCARDKRYSSGWYENWGQ
GTLVTVSSGGGGSGGGGSGGGASNFMLTQPHSVSESPGKTVTISCTRSSGSIASKYVQWYQQRPGSSPTTVIYEDNQ
RPSGVPDRFSGSIDISSNSASLTISGLKTEDEADYFCQSYDSSSVIFGGGTKLTVLGQPAAAGGGGSQVQLQQSGAE
LVRPGSSVKISCKASGYAFSSYWMNWVKQRPGQGLEWIGQIWPGDGDTNYNGKFKGKATLTADESSSTAYMQLSSLA
SEDSAVYFCARRETTTVGRYYYAMDYWGQGTSVTVSSASAAVECPPCPAPPVAGPSVFLFPPKPKDTLMISRTPEVT
CVVVDVSHEDPEVQFNWYVDGVEVHNAKTKPREEQFNSTFRVVSVLTVVHQDWLNGKEYKCKVSNKGLPAPIEKTIS
KTKGQPREPQVYTLPPSREEMTKNQVSLTCLVKGFYPSDIAVEWESNGQPENNYKTTPPMLDSDGSFFLYSKLTVDK
SRWQQGNVFSCSVMHEALHNHYTQKSLSLSPGK


<210> 6
<211> 722
<212> PRT
<213> Artificial sequence
<220>
<223> clone 309_04_A04
<400> 6

DIQLTQTPASLAVSLGQRATISCKASQSVDYDGDSYLNWYQQIPGQPPKLLIYDASNLVSGIPPRFSGSGSGTDFTL
NIHPVEKVDAATYHCQQSTEDPWTFGGGTKLEIKGGSGGAMAQVQLQQWGAGLLKPSETLSLTCAVYGGSFSGYYWS
WIRQPPGKGLEWIGEISRSGTTNYNSSLKSRVTISVDTSKNQFSLKLSSVTAADTAVYYCARATRNGVFDYWGQGTL
VTVSSGGGGSGGGGSGGGASNFMLTQPHSVSESPGKTVTISCTRSSGSIASNYVQWYQQRPGSAPSTVIYEDNQRPS
GVPDRFSGSIDSSSNSASLTISGLKTEDEADYYCQSYDSGVVFGGGTKLTVLGQPAAAGGGGSQVQLQQSGAELVRP
GSSVKISCKASGYAFSSYWMNWVKQRPGQGLEWIGQIWPGDGDTNYNGKFKGKATLTADESSSTAYMQLSSLASEDS
AVYFCARRETTTVGRYYYAMDYWGQGTSVTVSSASAAVECPPCPAPPVAGPSVFLFPPKPKDTLMISRTPEVTCVVV
DVSHEDPEVQFNWYVDGVEVHNAKTKPREEQFNSTFRVVSVLTVVHQDWLNGKEYKCKVSNKGLPAPIEKTISKTKG
QPREPQVYTLPPSREEMTKNQVSLTCLVKGFYPSDIAVEWESNGQPENNYKTTPPMLDSDGSFFLYSKLTVDKSRWQ
QGNVFSCSVMHEALHNHYTQKSLSLSPGK


<210> 7
<211> 726
<212> PRT
<213> Artificial sequence
<220>

<223> clone 309_05_B08
<400> 7

DIQLTQTPASLAVSLGQRATISCKASQSVDYDGDSYLNWYQQIPGQPPKLLIYDASNLVSGIPPRFSGSGSGTDFTL
NIHPVEKVDAATYHCQQSTEDPWTFGGGTKLEIKGGSGGAMAEVQLLESGGGLVQPGGSLRLSCAASGFTFSSYAMS
WVRQAPGKGLEWVSAISGSGGSTYYADSVKGRFTISRDNSKNTLYLQMNSLRAEDTAVYYCAISTVTRPDFDYWGQG
TLVTVSSGGGGSGGGGSGGGASNFMLTQPHSVSESPGKTVTISCTRSSGSIARNYVQWYQQRPGSAPTTVIYEDNQR
PSGVPDRFSGSIDSSSNSASLTISGLKTEDEADYYCQSYDSSNQRVFGGGTKVTVLGQPAAAGGGGSQVQLQQSGAE
LVRPGSSVKISCKASGYAFSSYWMNWVKQRPGQGLEWIGQIWPGDGDTNYNGKFKGKATLTADESSSTAYMQLSSLA
SEDSAVYFCARRETTTVGRYYYAMDYWGQGTSVTVSSASAAVECPPCPAPPVAGPSVFLFPPKPKDTLMISRTPEVT
CVVVDVSHEDPEVQFNWYVDGVEVHNAKTKPREEQFNSTFRVVSVLTVVHQDWLNGKEYKCKVSNKGLPAPIEKTIS
KTKGQPREPQVYTLPPSREEMTKNQVSLTCLVKGFYPSDIAVEWESNGQPENNYKTTPPMLDSDGSFFLYSKLTVDK
SRWQQGNVFSCSVMHEALHNHYTQKSLSLSPGK


<210> 8
<211> 729
<212> PRT
<213> Artificial sequence
<220>
<223> clone 400_01_D06
<400> 8

DIQLTQTPASLAVSLGQRATISCKASQSVDYDGDSYLNWYQQIPGQPPKLLIYDASNLVSGIPPRFSGSGSGTDFTL
NIHPVEKVDAATYHCQQSTEDPWTFGGGTKLEIKGGSGGAMAQVQLVQSGAEVKKPGSSVKVSCKASGGTFSSYAIS
WVRQAPGQGLEWMGGIIPIFGTANYAQKFQGRVTITADESTSTAYMEVRSLRSDDTAVYYCVRDRGDYGDITYFDYW
GQGTLVTVSSGGGGSGGGGSGGGASNFMLTQPHSVSESPGKTVTISCTGSSGSIASNYVQWYQQRPGSAPTIVIYED
NHRPSGVPDRFSGSIDSSSNSASLTISGLKTEDEADYYCQSYDSSDRVVFGGGTKVTVLGQPAAAGGGGSQVQLQQS
GAELVRPGSSVKISCKASGYAFSSYWMNWVKQRPGQGLEWIGQIWPGDGDTNYNGKFKGKATLTADESSSTAYMQLS
SLASEDSAVYFCARRETTTVGRYYYAMDYWGQGTSVTVSSASAAVECPPCPAPPVAGPSVFLFPPKPKDTLMISRTP
EVTCVVVDVSHEDPEVQFNWYVDGVEVHNAKTKPREEQFNSTFRVVSVLTVVHQDWLNGKEYKCKVSNKGLPAPIEK
TISKTKGQPREPQVYTLPPSREEMTKNQVSLTCLVKGFYPSDIAVEWESNGQPENNYKTTPPMLDSDGSFFLYSKLT
VDKSRWQQGNVFSCSVMHEALHNHYTQKSLSLSPGK


<210> 9
<211> 725
<212> PRT
<213> Artificial sequence
<220>
<223> clone 400_03_H07
<400> 9

DIQLTQTPASLAVSLGQRATISCKASQSVDYDGDSYLNWYQQIPGQPPKLLIYDASNLVSGIPPRFSGSGSGTDFTL
NIHPVEKVDAATYHCQQSTEDPWTFGGGTKLEIKGGSGGAMAQVQLVESGGGVVQPGRSLRLSCAASGFTFSSYSMN
WVRQAPGKGLEWVSSISSSSSYIYYADSVKGRFTISRDNAKNSLYLQMNSLRAEDTAVYYCARSGIAAAGFDYWGQG
TLVTVSSGGGGSGGGGSGGGASNFMLTQPHSVSESPGKTVTISCARSSGSIASNYVQWYQQRPGSSPTTVIYEDNRR
PSGVPDRFSGSFDSSSNSASLTISGLKTEDEADYYCQSYDSSNRVFGGGTKLTVLGQPAAAGGGGSQVQLQQSGAEL
VRPGSSVKISCKASGYAFSSYWMNWVKQRPGQGLEWIGQIWPGDGDTNYNGKFKGKATLTADESSSTAYMQLSSLAS
EDSAVYFCARRETTTVGRYYYAMDYWGQGTSVTVSSASAAVECPPCPAPPVAGPSVFLFPPKPKDTLMISRTPEVTC
VVVDVSHEDPEVQFNWYVDGVEVHNAKTKPREEQFNSTFRVVSVLTVVHQDWLNGKEYKCKVSNKGLPAPIEKTISK
TKGQPREPQVYTLPPSREEMTKNQVSLTCLVKGFYPSDIAVEWESNGQPENNYKTTPPMLDSDGSFFLYSKLTVDKS
RWQQGNVFSCSVMHEALHNHYTQKSLSLSPGK


<210> 10
<211> 723
<212> PRT
<213> Artificial sequence
<220>
<223> clone 410_01_C03
<400> 10

DIQLTQTPASLAVSLGQRATISCKASQSVDYDGDSYLNWYQQIPGQPPKLLIYDASNLVSGIPPRFSGSGSGTDFTL
NIHPVEKVDAATYHCQQSTEDPWTFGGGTKLEIKGGSGGAMAQVQLQQWGAGLLKPSETLSLTCAVYGGSFSGYYWS
WIRQPPGKGLEWIGEINHSGSTNYNPSLKSRVTISVDTSKNQFSLKLSSVTAADTAVYYCARATRNGAFDYWGQGTL

VTVSSGGGGSGGGGSGGGASNFMLTQPHSVSESPGKTVTISCTRSSGRIASNYVQWYQQRPGSSPTTVIYEDNQRPS
GVPDRFSGSIDSSSNSASLTISGLKTEDEADYYCQSYDSSNVVFGGGTQLTVLGQPAAAGGGGSQVQLQQSGAELVR
PGSSVKISCKASGYAFSSYWMNWVKQRPGQGLEWIGQIWPGDGDTNYNGKFKGKATLTADESSSTAYMQLSSLASED
SAVYFCARRETTTVGRYYYAMDYWGQGTSVTVSSASAAVECPPCPAPPVAGPSVFLFPPKPKDTLMISRTPEVTCVV
VDVSHEDPEVQFNWYVDGVEVHNAKTKPREEQFNSTFRVVSVLTVVHQDWLNGKEYKCKVSNKGLPAPIEKTISKTK
GQPREPQVYTLPPSREEMTKNQVSLTCLVKGFYPSDIAVEWESNGQPENNYKTTPPMLDSDGSFFLYSKLTVDKSRW
QQGNVFSCSVMHEALHNHYTQKSLSLSPGK


<210> 11
<211> 725
<212> PRT
<213> Artificial sequence
<220>
<223> clone 410_01_C09
<400> 11

DIQLTQTPASLAVSLGQRATISCKASQSVDYDGDSYLNWYQQIPGQPPKLLIYDASNLVSGIPPRFSGSGSGTDFTL
NIHPVEKVDAATYHCQQSTEDPWTFGGGTKLEIKGGSGGAMAEVQLVQSGAEVKKPGASVKVSCKASGYTFTSYYMY
WVRQAPGQGLEWMGRINPNSGGTNYAQKFQGRVTMTRDTSISTAYMELSRLRSDDTAVYYCARPSRYSNYVDWGQGT
LVTVSSGGGGSGGGGSGGGASNFMLTQPHSVSESPGKTVTISCTRSSGSIASNYVQWYQQRPGSSPTTVIYEDNQRP
SGVPDRFSGSIDSSSNSASLTISGLKTEDEADYYCQSYDSRWIWVSGGGTKVTVLGQPAAAGGGGSQVQLQQSGAEL
VRPGSSVKISCKASGYAFSSYWMNWVKQRPGQGLEWIGQIWPGDGDTNYNGKFKGKATLTADESSSTAYMQLSSLAS
EDSAVYFCARRETTTVGRYYYAMDYWGQGTSVTVSSASAAVECPPCPAPPVAGPSVFLFPPKPKDTLMISRTPEVTC
VVVDVSHEDPEVQFNWYVDGVEVHNAKTKPREEQFNSTFRVVSVLTVVHQDWLNGKEYKCKVSNKGLPAPIEKTISK
TKGQPREPQVYTLPPSREEMTKNQVSLTCLVKGFYPSDIAVEWESNGQPENNYKTTPPMLDSDGSFFLYSKLTVDKS
RWQQGNVFSCSVMHEALHNHYTQKSLSLSPGK


<210> 12
<211> 728
<212> PRT
<213> Artificial sequence
<220>
<223> clone 418_01_C05
<400> 12

DIQLTQTPASLAVSLGQRATISCKASQSVDYDGDSYLNWYQQIPGQPPKLLIYDASNLVSGIPPRFSGSGSGTDFTL
NIHPVEKVDAATYHCQQSTEDPWTFGGGTKLEIKGGSGGAMAEVQLVESGGGVVQPGRSLRLSCAASGFTFSSYGMH
WVRQAPGKGLEWVSYINSRSSTIYYADSVKGRFTISRDNAKNSLYLQMNSLRDEDTAVYYCAKDKPLSGYAFDIWGQ
GTTVTVSSGGGGSGGGGSGGGASNFMLTQPHSVSESPGKTVTISCTRSSGSIASNFVQWYQQRPGRAPTIVIFDDDQ
RPSGVPDRFSGSIDRSSDSASLSISGLKTEDEADYYCQSYATRTQGVVFGGGTKVTVLGQPAAAGGGGSQVQLQQSG
AELVRPGSSVKISCKASGYAFSSYWMNWVKQRPGQGLEWIGQIWPGDGDTNYNGKFKGKATLTADESSSTAYMQLSS
LASEDSAVYFCARRETTTVGRYYYAMDYWGQGTSVTVSSASAAVECPPCPAPPVAGPSVFLFPPKPKDTLMISRTPE
VTCVVVDVSHEDPEVQFNWYVDGVEVHNAKTKPREEQFNSTFRVVSVLTVVHQDWLNGKEYKCKVSNKGLPAPIEKT
ISKTKGQPREPQVYTLPPSREEMTKNQVSLTCLVKGFYPSDIAVEWESNGQPENNYKTTPPMLDSDGSFFLYSKLTV
DKSRWQQGNVFSCSVMHEALHNHYTQKSLSLSPGK


<210> 13
<211> 727
<212> PRT
<213> Artificial sequence
<220>
<223> clone 418_03_E12
<400> 13

DIQLTQTPASLAVSLGQRATISCKASQSVDYDGDSYLNWYQQIPGQPPKLLIYDASNLVSGIPPRFSGSGSGTDFTL
NIHPVEKVDAATYHCQQSTEDPWTFGGGTKLEIKGGSGGAMAQVQLVESGGGVVQPGRSLRLSCAASGFTFSSYGMH
WVRQAPGKGLEWVSSISSSSSYIYYADSVKGRFTISRDNAKNSLYLQMNSLRADDTAVYYCARRSFSSVFADYWGQG
TLVTVSSGGGGSGGGGSGGGASNFMLTQPHSVSESPGKTVTISCTRSRGSIASNYVQWYQQRPGRAPTIVIFDDDQR
PSGVPDRFSGSIDRSSDSASLRISGLKTEDEADYYCQSYATRSQSVVFGGGTKLTVLGQPAAAGGGGSQVQLQQSGA
ELVRPGSSVKISCKASGYAFSSYWMNWVKQRPGQGLEWIGQIWPGDGDTNYNGKFKGKATLTADESSSTAYMQLSSL
ASEDSAVYFCARRETTTVGRYYYAMDYWGQGTSVTVSSASAAVECPPCPAPPVAGPSVFLFPPKPKDTLMISRTPEV
TCVVVDVSHEDPEVQFNWYVDGVEVHNAKTKPREEQFNSTFRVVSVLTVVHQDWLNGKEYKCKVSNKGLPAPIEKTI
SKTKGQPREPQVYTLPPSREEMTKNQVSLTCLVKGFYPSDIAVEWESNGQPENNYKTTPPMLDSDGSFFLYSKLTVD

KSRWQQGNVFSCSVMHEALHNHYTQKSLSLSPGK

```
<210>   14
<211>   723
<212>   PRT
<213>   Artificial sequence
<220>
<223>   clone 418_03_H01
<400>   14
```

DIQLTQTPASLAVSLGQRATISCKASQSVDYDGDSYLNWYQQIPGQPPKLLIYDASNLVSGIPPRFSGSGSGTDFTL
NIHPVEKVDAATYHCQQSTEDPWTFGGGTKLEIKGGSGGAMAQVQLQQWGAGLLKPSETLSLTCAVYGGSFSRYYWS
WIRQSPGKGLEWIGEIKHRGRTNYNPSLKSRVTMSVDTSKNQFSLQLRSVTAADTAVYYCARVYSSGWFSYWGQGTL
VTVSSGGGGSGGGGSGGGASNFMLTQPHSVSESPGKTVTISCTRSSGSIARNYVQWYQQRPGSAPTTVIYEDNQRPS
GVPDRFSGSIDSSSNSASLTISGLKTEDEADYYCQSYDGSNWVFGGGTKVTVLGQPAAAGGGGSQVQLQQSGAELVR
PGSSVKISCKASGYAFSSYWMNWVKQRPGQGLEWIGQIWPGDGDTNYNGKFKGKATLTADESSSTAYMQLSSLASED
SAVYFCARRETTTVGRYYYAMDYWGQGTSVTVSSASAAVECPPCPAPPVAGPSVFLFPPKPKDTLMISRTPEVTCVV
VDVSHEDPEVQFNWYVDGVEVHNAKTKPREEQFNSTFRVVSVLTVVHQDWLNGKEYKCKVSNKGLPAPIEKTISKTK
GQPREPQVYTLPPSREEMTKNQVSLTCLVKGFYPSDIAVEWESNGQPENNYKTTPPMLDSDGSFFLYSKLTVDKSRW
QQGNVFSCSVMHEALHNHYTQKSLSLSPGK

```
<210>   15
<211>   7
<212>   PRT
<213>   Artificial sequence
<220>
<223>   hinge region
<400>   15
```

VECPPCP

```
<210>   16
<211>   216
<212>   PRT
<213>   Homo sapiens
<220>
<223>   Fc-fragment of human IgG2
<400>   16
```

APPVAGPSVFLFPPKPKDTLMISRTPEVTCVVVDVSHEDPEVQFNWYVDGVEVHNAKTKPREEQFNSTFRVVSVLTV
VHQDWLNGKEYKCKVSNKGLPAPIEKTISKTKGQPREPQVYTLPPSREEMTKNQVSLTCLVKGFYPSDIAVEWESNG
QPENNYKTTPPMLDSDGSFFLYSKLTVDKSRWQQGNVFSCSVMHEALHNHYTQKSLSLSPGK

```
<210>   17
<211>   8
<212>   PRT
<213>   Artificial sequence
<220>
<223>   linker 1
<400>   17
```

GGSGGAMA

```
<210>   18
<211>   15
<212>   PRT
<213>   Artificial sequence
<220>
<223>   linker 2
<400>   18
```

GGGGSGGGGSGGGAS

```
<210>   19
<211>   8
<212>   PRT
<213>   Artificial sequence
<220>
<223>   linker 3
<400>   19

AAAGGGGS
```

## Claims

1. A protein molecule capable of binding a CD3 receptor complex, comprising at least one antigen-binding fragment comprising a light chain variable domain and a heavy chain variable domain, **characterized in that** heavy-chain complementarity-determining regions HCDR1, HCDR2 and HCDR3 and light-chain complementarity-determining regions LCDR1, LCDR2 and LCDR3 are selected from the group composed of variants 1-14 shown in Table 2.

2. The protein molecule of Claim 1, which monovalently and specifically binds CD3 located at human T-lymphocytes.

3. The protein molecule of Claim 1, wherein LCDR1, LCDR2 and LCDR3, contained in a light chain variable domain specific to CD3, are identical to LCDR1, LCDR2 and LCDR3 contained in a sequence of an antibody selected from the following list: >309_01_A02; >309_01_G04; >309_01_G12; >309_02_A08; >309_02_F01; >309_04_A04; >309_05_B08; >400_01_D06; >400_03_H07; >410_01_C03;>410_01_C09; >418_01_C05; >418_03_E12; >418_03_H01.

4. The protein molecule of Claim 1, wherein HCDR1, HCDR2 and HCDR3, contained in a heavy chain variable domain specific to CD3, are identical to HCDR1, HCDR2 and HCDR3 contained in a sequence of an antibody selected from the following list: >309_01_A02; >309_01_G04; >309_01_G12; >309_02_A08; >309_02_F01; >309_04_A04; >309_05_B08; >400_01_D06; >400_03_H07; >410_01_C03;>410_01_C09; >418_01_C05; >418_03_E12; >418_03_H01.

5. The protein molecule of any one of Claims 1-4, which forms a part of a bispecific antigen-binding molecule.

6. The protein molecule of any one of Claims 1-5, which contains a second antigen-binding polypeptide construct that monovalently and specifically binds CD19 located on human B-lymphocytes.

7. The protein molecule of any one of Claims 1-6, which sequence comprises: (a) a first domain comprising an antigen-binding region of the molecule of any one of Claims 1-5, and (b) a second domain comprising an antigen-binding region of an immunoglobulin or antibody chain that specifically recognizes an antigen directed against CD19 of human B-cells, the domains forming said antigen-binding regions being arranged in the following order: VLCD3-VHCD19-VHCD19-VLCD3.

8. The protein molecule of any one of Claims 1-7, which further comprises a Fc-fragment.

9. The protein molecule of any one of Claims 1-8, which is capable of inducing activation of T-cells in a bispecific antibody format.

10. The protein molecule of any one of Claims 1-9, which amino acid sequence comprises sequences selected from the following list: SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14.

11. Use of the molecule of any one of Claims 1-10 for creating a medicine intended for treating B-cell malignancies or for depleting B-cells.

12. The use of Claim 11, where said medicine comprises the molecule of any one of Claims 1-10 in an effective quantity and, optionally, neutral carriers from a range of carriers traditionally used in compositions of this purpose.

**13.** The use of Claim 11, where said B-cell malignancy is selected from acute lymphoblastic leukemia and B-cell lymphomas.

**14.** The use of Claim 13, where said diseases progresses in a refractory or recurrent form.

Fig. 1A

| Bottom | 1.015 |
| Top | 30.71 |
| LogEC50 | -0.5041 |
| HillSlope | 1.149 |
| EC50 | 0.3132 |
| R square | 0.9858 |

Fig. 1B

| Top | 15.46 |
| LogEC50 | -0.3515 |
| HillSlope | 0.7472 |
| EC50 | 0.4451 |
| R square | 0.9817 |

| Bottom | 1.525 |
|--------|-------|
| Top | 59.87 |
| LogEC50 | -0.4897 |
| HillSlope | 1.185 |
| EC50 | 0.3238 |
| R square | 0.9912 |

C

Fig. 1C

| Bottom | 1.626 |
|--------|-------|
| Top | 52.06 |
| LogEC50 | -0.4560 |
| HillSlope | 1.398 |
| EC50 | 0.3499 |
| R square | 0.9807 |

D

Fig. 1D

| Bottom | 18.41 |
|---|---|
| Top | 286.8 |
| LogEC50 | -0.6279 |
| HillSlope | 1.408 |
| EC50 | 0.2355 |
| R square | 0.9988 |

E

Fig. 1E

F

| Bottom | 23.92 |
|---|---|
| Top | 390.6 |
| LogEC50 | -0.4314 |
| HillSlope | 1.571 |
| EC50 | 0.3704 |
| R square | 0.9947 |

Fig. 1F

| Bottom | 4.481 |
| Top | 139.8 |
| LogEC50 | 0.07901 |
| HillSlope | 1.394 |
| EC50 | 1.200 |
| R square | 0.9963 |

Fig. 2A

| Bottom | 1.217 |
| Top | 4.610 |
| LogEC50 | -0.2848 |
| HillSlope | 1.151 |
| EC50 | 0.5191 |
| R square | 0.9568 |

Fig. 2B

C

| Bottom | 12.55 |
|---|---|
| Top | 148.8 |
| LogEC50 | -0.4058 |
| HillSlope | 1.816 |
| EC50 | 0.3928 |
| R square | 0.9871 |

Fig. 2C

| | Donor 1 | Donor 2 | Donor 3 |
|---|---|---|---|
| Bottom | 640.4 | 565.4 | 655.9 |
| Top | 1301 | 1195 | 1276 |
| LogIC50 | -1.289 | -1.364 | -1.312 |
| HillSlope | -1.188 | -1.012 | -1.739 |
| IC50 | 0.05136 | 0.04324 | 0.04871 |
| | Donor 1 | Donor 2 | Donor 3 |
| R square | 0.9917 | 0.9742 | 0.9535 |

Fig. 3

| | Donor 1 | Donor 2 | Donor 3 |
|---|---|---|---|
| Bottom | 99.15 | 947.5 | 147.8 |
| Top | 16984 | 11858 | 14844 |
| LogIC50 | 1.823 | 1.717 | 1.794 |
| HillSlope | -1.023 | -1.023 | -0.9638 |
| IC50 | 66.47 | 52.06 | 62.28 |

| | Donor 1 | Donor 2 | Donor 3 |
|---|---|---|---|
| R square | 0.9967 | 0.9917 | 0.9982 |

Fig. 4

| | Donor 1 | Donor 2 | Donor 3 | Donor 4 | Donor 5 |
|---|---|---|---|---|---|
| Bottom | 1.119 | 1.051 | -0.01028 | 0.7708 | 2.884 |
| Top | 59.46 | 85.92 | 55.00 | 22.22 | 96.41 |
| LogEC50 | -1.031 | -1.385 | -0.9967 | -0.9466 | -1.070 |
| HillSlope | 0.9845 | 1.129 | 1.057 | 1.162 | 0.7238 |
| EC50 | 0.09304 | 0.04120 | 0.1008 | 0.1131 | 0.08517 |

| | Donor 1 | Donor 2 | Donor 3 | Donor 4 | Donor 5 |
|---|---|---|---|---|---|
| R square | 0.9958 | 0.9976 | 0.9965 | 0.9935 | 0.9873 |

Fig. 5

Fig. 6

Fig. 7

Fig. 8

| | BiMS2 | 309-02-A08 |
|---|---|---|
| Bottom | 3344 | 3344 |
| Top | 93279 | 93279 |
| LogEC50 | 1.076 | 0.2445 |
| HillSlope | 0.7529 | 0.7529 |
| EC50 | 11.92 | 1.756 |
| | BiMS2 | 309-02-A08 |
| R square | 0.9599 | 0.9552 |

Fig. 9

| | G04 |
|---|---|
| EC50 | 0.7918 |

| | G04 |
|---|---|
| R square | 0.9857 |

Fig. 10(A)

| | A02 | Global (shared) |
|---|---|---|
| EC50 | 2.961 | |

| | A02 | Global (shared) |
|---|---|---|
| R square | 0.9896 | 0.9896 |

Fig. 10(B)

| | F01 | Global (shared) |
|---|---|---|
| EC50 | 3.196 | |

| | F01 | Global (shared) |
|---|---|---|
| R square | 0.9796 | 0.9796 |

Fig. 10(C)

| | G12 | Global (shared) |
|---|---|---|
| EC50 | 2.692 | |

| | G12 | Global (shared) |
|---|---|---|
| R square | 0.9685 | 0.9685 |

Fig. 10(D)

| | A08 | Global (shared) |
|---|---|---|
| EC50 | 0.03454 | |

| | A08 | Global (shared) |
|---|---|---|
| R square | 0.9395 | 0.9395 |

Fig. 10(E)

| | B08 | Global (shared) |
|---|---|---|
| EC50 | 8.390 | |

| | B08 | Global (shared) |
|---|---|---|
| R square | 0.9835 | 0.9835 |

Fig. 10(F)

**Chain 1**            **Chain 2**

Fig. 11

**INTERNATIONAL SEARCH REPORT**

| International application No. |
| --- |
| PCT/RU 2020/050195 |

| A. CLASSIFICATION OF SUBJECT MATTER | |
| --- | --- |
| *C07K 16/28* (2006.01)    *A61K 39/395* (2006.01) | |
| *C07K 16/46* (2006.01)    *A61P 35/*00 (2006.01) | |
| According to International Patent Classification (IPC) or to both national classification and IPC | |

| B. FIELDS SEARCHED |
| --- |
| Minimum documentation searched (classification system followed by classification symbols) |
| C07K, A61K, A61P |
| Documentation searched other than minimum documentation to the extent that such documents are included in the fields searched |
| |
| Electronic data base consulted during the international search (name of data base and, where practicable, search terms used) |
| Patentscope, Espacenet, USPTO, RUPAT, EAPATIS, CIPO, KIPRIS, K-PION, SIPO, DWPI; MedLine, PubMed Central, Scopus, EMBASE, РИНЦ (e-Library) |

| C. DOCUMENTS CONSIDERED TO BE RELEVANT | | |
| --- | --- | --- |
| Category* | Citation of document, with indication, where appropriate, of the relevant passages | Relevant to claim No. |
| Y | WO 2014/047231 A1 (REGENERON PHARMACEUTICALS, INC.), 27.03.2014, the claims, [0069], [0070], [0072], [0094], [0101], [0115], [0125], [0157], example 10 | 1-6, 11-14 |
| Y | EP 2686347 A1 (ARGEN X BV), 22.01.2014, the claims | 1-6, 11-14 |
| Y | EP 2640416 A1 (MEDIMMUNE LLC [US]), 25.09.2013, the claims, p.16 last paragraph -p.17, p.26 last paragraph -p.27 second paragraph, p.29 | 11-14 |

| ☐ Further documents are listed in the continuation of Box C. | ☐ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 15 December 2020 (15.12.2020) | 14 January 2021 (14.01.2021) |
| Name and mailing address of the ISA/ RU | Authorized officer |
| Facsimile No. | Telephone No. |

Form PCT/ISA/210 (second sheet) (January 2015)

**INTERNATIONAL SEARCH REPORT**

| International application No. |
| --- |
| PCT/RU 2020/050195 |

---

**Box No. II    Observations where certain claims were found unsearchable (Continuation of item 2 of first sheet)**

This international search report has not been established in respect of certain claims under Article 17(2)(a) for the following reasons:

1. [X] Claims Nos.: 7-10
   because they relate to subject matter not required to be searched by this Authority, namely:

2. [ ] Claims Nos.:
   because they relate to parts of the international application that do not comply with the prescribed requirements to such an extent that no meaningful international search can be carried out, specifically:

3. [X] Claims Nos.: 7-10
   because they are dependent claims and are not drafted in accordance with the second and third sentences of Rule 6.4(a).

---

**Box No. III    Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying additional fees, this Authority did not invite payment of additional fees.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**
[ ] The additional search fees were accompanied by the applicant's protest and, where applicable, the payment of a protest fee.
[ ] The additional search fees were accompanied by the applicant's protest but the applicable protest fee was not paid within the time limit specified in the invitation.
[ ] No protest accompanied the payment of additional search fees.

Form PCT/ISA/210 (continuation of first sheet (2)) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- RU 2228202
- RU 2651776
- RU 2613368

**Non-patent literature cited in the description**

- **DEYEV S.M. ; LEBEDENKO YE.N.** Modern technologies for creating non-natural antibodies for clinical use. *Acta Naturae (Russian-language version),* 2009, vol. 1 (1), 32-50
- *Blood, (ASH Annual Meeting Abstracts),* 2009, vol. 114, 840
- **HMILA I. et al.** *Mol. Immunol. England,* 2008, vol. 45 (14), 3847-3856